# EUROPEAN PATENT APPLICATION

(11) **EP 4 317 175 A1**
(43) Date of publication of application: **07.02.2024**
(21) Application number: 22779054.0
(22) Date of filing: 31.03.2022
(51) Int. Cl.: C07K 14/00, C07K 19/00, C12N 15/62, A61P 37/02

(54) **TRUNCATED TACI POLYPEPTIDE AND FUSION PROTEIN AND USE THEREOF**

(30) Priority: 31.03.2021 CN 202110348497
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: MAO, Langyong, Shanghai 200245 (CN); YING, Hua, Shanghai 200245 (CN); JIN, Xinsheng, Shanghai 200245 (CN); LI, Lingling, Shanghai 200245 (CN); TAO, Weikang, Shanghai 200245 (CN)
(74) Representative: Dragotti & Associati S.R.L.
(86) International application number: PCT/CN2022/084256
(87) International publication number: WO 2022/206872

(57) **Abstract**

The present application relates to a truncated TACI polypeptide and a fusion protein and use thereof. Specifically, provided are a TACI polypeptide as shown in SEQ ID NO: 8, a truncated fragment thereof, a mutation sequence thereof, and a fusion protein comprising the TACI polypeptide and use thereof.

## Description

The present application claims priority to Chinese Patent Application No. 202110348497.6 filed on Mar. 31, 2021, which is incorporated herein by reference in its entirety.

### TECHNICAL FIELD

The present disclosure belongs to the field of biotechnology and particularly relates to a novel TACI polypeptide, and a fusion protein and use thereof.

### BACKGROUND

The statements herein merely provide background information related to the present disclosure and may not necessarily constitute the prior art.

Systemic lupus erythematosus (SLE) is an autoimmune disease that affects multiple organs. A variety of cells are involved in the development and progression of SLE (Tsokos, G.C., et al., Nat Rev Rheumatol, 2016. 12: p. 716-730; Larosa, M. et al., Expert Rev Clin Immunol, 2016. 12: p. 1309-1320). Neutrophils release a reticular extracellular trap that captures antibacterial proteins and in conjunction with interferon, stimulates dendritic cells to secrete a large amount of type I interferon. The interferon further activates myeloid dendritic cells to produce a variety of cytokines that activate T cells or B cells. IL-12 and IL-23 are two cytokines that are known to activate T cell differentiation. IL-12/IL-23 can stimulate T cells to secrete IL-17 and IL-21. IL-17 works with cytokines secreted by macrophages, such as TNF and IL-6, to recruit inflammatory cells and cause tissue damage. In contrast, IL-21 can co-stimulate B cells to participate in regulating the proliferation of B cells. BAFF is an activator of B cells. BAFF stimulates the maturation and survival of B cells by interacting with receptors on the surface of B cells, and B cells eventually differentiate into plasma cells and secrete autoantibodies under the combined action of multiple cytokines. The autoantibodies accumulate around various tissues and organs, causing damage to the tissue and organs (Murphy, G. and D.A. Isenberg, Nat Rev Rheumatol, 2019. 15: p. 403-412; Lam, N.C., M.V. Ghetu, and M.L. Bieniek, Am Fam Physician, 2016. 94: p. 284-294).

IL-12 and IL-23 are two cytokines that belong to the same family. Both IL-12 and IL-23 are heterodimeric proteins consisting of two subunits (Moschen, A.R., H. Tilg, and T. Raine, Nat Rev Gastroenterol Hepatol, 2019. 16: p. 185-196; Frieder, J., et al., Clin Pharmacol Ther, 2018. 103: p. 88-101). IL-12 consists of two subunits, p35 and p40, whereas IL-23 consists of two subunits, p19 and p40. p40 is a subunit shared by them, so antibodies targeting p40 can inhibit both IL-12 and IL-23 signaling pathways. IL-12 interacts with receptors to mainly activate the differentiation of Th1 cells and is also involved in stimulating the secretion of interferon γ and TNF by a variety of immune cells. IL-23 interacts with receptors to mainly activate the differentiation of Th17 cells, and stimulates the secretion of cytokines such as IL-17, IL-22, and TNF by a variety of cells (Lee, E.B., et al., Cutis, 2018. 101: p. 5-9; Floss, D.M., et al., Cytokine Growth Factor Rev, 2015. 26: p. 569-578). These cytokines produced by the activation of the IL-12/IL-23 signaling pathway are further involved in the progression of SLE disease through their respective pathways.

TACI is a membrane-bound receptor with an extracellular region comprising two cysteine-rich pseudo-repeats, a transmembrane region, and a cytoplasmic region that interacts with CAML (calcium-modulating cyclophilin ligand). TACI is associated with one subtype of B cells and T cells. TACI receptors bind to BAFF of the tumor necrosis factor ligand family. BAFF is a B cell activator that belongs to the TNF family. BAFF is mainly expressed on the cell membrane surface of bone marrow cells and exists in the form of trimer. BAFF on the cell membrane surface is hydrolyzed by a protease to form soluble BAFF that enters the blood circulation system. Soluble BAFF is characterized by multimerization and can form at most a 60-mer. In addition, BAFF can also interact with another protein of the same family, APRIL, to form a heterotrimer. Three receptors for BAFF, i.e., BAFF-R, BCMA, and TACI, are currently known on the surface of B cells. BAFF interacts with these three receptors and is involved in the differentiation maturation, survival, and regulation of B cells. APRIL shares two receptors with BAFF, i.e., BCMA and TACI, and functions with these two receptors to participate in the survival and regulation of B cells (Samy, E., et al., Int Rev Immunol, 2017. 36: p. 3-19; Kamal, A. and M. Khamashta, Autoimmun Rev, 2014. 13: p. 1094-1101). BAFF is important for maintaining B cell homeostasis, and over-activation of the BAFF signaling pathway leads to the survival of autoreactive B cells and the production of autoantibodies that promote autoimmune responses (Cancro, M.P., D.P. D'Cruz, and M.A. Khamashta, J Clin Invest, 2009. 119: p. 1066-73).

### SUMMARY

Due to the presence of multiple protease digestion sites in the sequence of TACI, full-length TACI is very susceptible to cleavage after expression. In order to solve the problem of TACI cleavage, in the present disclosure, a novel TACI sequence fragment is designed and amino acid residues at part of positions are replaced to construct a novel TACI polypeptide and a fusion protein thereof.

The present disclosure provides a TACI polypeptide, which has a sequence set forth in SEQ ID NO: 8 or is a truncated fragment of SEQ ID NO: 8 or a variant thereof, wherein the truncated fragment comprises amino acid residues at positions 51-85 of SEQ ID NO: 8, and the variant is a variant having one or more amino acid replacements at positions selected from the group consisting of positions 52, 53, 57, 65, 82, and 83 in SEQ ID NO: 8 or the truncated fragment thereof, wherein the positions for the amino acid replacements are amino acid residue positions numbered in natural order relative to the sequence SEQ ID NO: 8.

In one aspect, the present disclosure provides a TACI polypeptide, which has a sequence set forth in SEQ ID NO: 8 or is a truncated fragment of SEQ ID NO: 8 or a variant thereof, wherein the truncated fragment comprises amino acid residues at positions 48-85 of SEQ ID NO: 8, and the variant is a variant having one or more amino acid replacements at positions selected from the group consisting of positions 49, 52, 53, 57, 65, 82, and 83 in SEQ ID NO: 8 or the truncated fragment thereof, wherein the positions for the amino acid replacements are amino acid residue positions numbered in natural order relative to the sequence SEQ ID NO: 8.

In some embodiments, provided is the TACI polypeptide described above, wherein the truncated fragment of the TACI polypeptide comprises: amino acid residues at positions 48-86 of SEQ ID NO: 8; amino acid residues at positions 48-87 of SEQ ID NO: 8; or amino acid residues at positions 48-88 of SEQ ID NO: 8.

In some embodiments, the truncated fragment of the TACI polypeptide comprises: amino acid residues at positions 50-85 of SEQ ID NO: 8; or amino acid residues at positions 49-85 of SEQ ID NO: 8.

In some embodiments, the TACI polypeptide described above has a sequence set forth in SEQ ID NO: 10, SEQ ID NO: 13, SEQ ID NO: 14, or SEQ ID NO: 15.

In some embodiments, provided is the TACI polypeptide, which has a sequence set forth in SEQ ID NO: 8 or is a variant of a truncated fragment (e.g., SEQ ID NO: 10, SEQ ID NO: 13, SEQ ID NO: 14, or SEQ ID NO: 15) of SEQ ID NO: 8, wherein the variant is a variant having any 1, 2, 3, 4, 5, 6, or 7 amino acid replacements at positions selected from the group consisting of positions 49, 52, 53, 57, 65, 82, and 83 in the sequence of SEQ ID NO: 8 or the truncated fragment thereof, wherein the positions for the amino acid replacements are amino acid residue positions numbered in natural order relative to the sequence SEQ ID NO: 8.

In some embodiments, provided is the TACI polypeptide described above, wherein the TACI polypeptide variant is a variant having one or more amino acid replacements (e.g., 1, 2, 3, 4, 5, 6, or 7 amino acid replacements) selected from the group consisting of 49T or 49R, 52S, 53E or 53Q, 57E, 65T or 65A, 82A or 82R, and 83Y in a sequence of SEQ ID NO: 8, SEQ ID NO: 10, SEQ ID NO: 13, SEQ ID NO: 14, or SEQ ID NO: 15, wherein the positions for the amino acid replacements are amino acid residue positions numbered in natural order relative to the sequence SEQ ID NO: 8.

In some embodiments, the TACI polypeptide variant is a variant having one or more amino acid replacements (e.g., 1, 2, 3, 4, 5, 6, or 7 amino acid replacements) selected from the group consisting of 49T or 49R, 52S, 53E or 53Q, 57E, 65T or 65A, 82A or 82R, and 83Y in a sequence of SEQ ID NO: 68, wherein the positions for the amino acid replacements are amino acid residue positions numbered in natural order relative to the sequence SEQ ID NO: 8.

In some embodiments, the TACI polypeptide variant is a variant having one or more amino acid replacements (e.g., 1, 2, 3, 4, 5, or 6 amino acid replacements) selected from the group consisting of 52S, 53E or 53Q, 57E, 65T or 65A, 82A or 82R, and 83Y in a sequence of SEQ ID NO: 69 or SEQ ID NO: 70, wherein the positions for the amino acid replacements are amino acid residue positions numbered in natural order relative to the sequence SEQ ID NO: 8. In some embodiments, the TACI polypeptide variant described above is selected from the group consisting of:
a variant having any one amino acid replacement selected from the group consisting of 49T, 52S, 53E, 53Q, 57E, and 82A in a sequence of SEQ ID NO: 8, SEQ ID NO: 10, SEQ ID NO: 13, SEQ ID NO: 14, or SEQ ID NO: 15;
a variant having amino acid replacements of 49R and 65T in a sequence of SEQ ID NO: 8, SEQ ID NO: 10, SEQ ID NO: 13, SEQ ID NO: 14, or SEQ ID NO: 15;
a variant having amino acid replacements of 49R and 65A in a sequence of SEQ ID NO: 8, SEQ ID NO: 10, SEQ ID NO: 13, SEQ ID NO: 14, or SEQ ID NO: 15;
a variant having amino acid replacements of 49R, 65T, and 82R in a sequence of SEQ ID NO: 8, SEQ ID NO: 10, SEQ ID NO: 13, SEQ ID NO: 14, or SEQ ID NO: 15;
a variant having amino acid replacements of 53E and 57E in a sequence of SEQ ID NO: 8, SEQ ID NO: 10, SEQ ID NO: 13, SEQ ID NO: 14, or SEQ ID NO: 15;
a variant having amino acid replacements of 52S, 53E, and 57E in a sequence of SEQ ID NO: 8, SEQ ID NO: 10, SEQ ID NO: 13, SEQ ID NO: 14, or SEQ ID NO: 15;
a variant having amino acid replacements of 49T and 82A in a sequence of SEQ ID NO: 8, SEQ ID NO: 10, SEQ ID NO: 13, SEQ ID NO: 14, or SEQ ID NO: 15;
a variant having amino acid replacements of 49T and 83Y in a sequence of SEQ ID NO: 8, SEQ ID NO: 10, SEQ ID NO: 13, SEQ ID NO: 14, or SEQ ID NO: 15;
a variant having amino acid replacements of 49T, 82A, and 83Y in a sequence of SEQ ID NO: 8, SEQ ID NO: 10, SEQ ID NO: 13, SEQ ID NO: 14, or SEQ ID NO: 15; or
a variant having amino acid replacements of 49T, 53E, 57E, and 82A in a sequence of SEQ ID NO: 8, SEQ ID NO: 10, SEQ ID NO: 13, SEQ ID NO: 14, or SEQ ID NO: 15;
wherein the positions for the amino acid replacements described above are amino acid residue positions numbered in natural order relative to the sequence SEQ ID NO: 8.

In some embodiments, the TACI polypeptide variant described above is selected from the group consisting of:
a variant having any one amino acid replacement selected from the group consisting of 49T, 52S, 53E, 53Q, 57E, and 82A in a sequence of SEQ ID NO: 68;
a variant having any one amino acid replacement selected from the group consisting of 52S, 53E, 53Q, 57E, and 82A in a sequence of SEQ ID NO: 69 or SEQ ID NO: 70;
a variant having amino acid replacements of 49R and 65T in a sequence of SEQ ID NO: 68;
a variant having amino acid replacements of 49R and 65A in a sequence of SEQ ID NO: 68;
a variant having amino acid replacements of 49R, 65T, and 82R in a sequence of SEQ ID NO: 68;
a variant having amino acid replacements of 53E and 57E in a sequence of SEQ ID NO: 68, SEQ ID NO: 69, or SEQ ID NO: 70;
a variant having amino acid replacements of 52S, 53E, and 57E in a sequence of SEQ ID NO: 68, SEQ ID NO: 69, or SEQ ID NO: 70;
a variant having amino acid replacements of 49T and 82A in a sequence of SEQ ID NO: 68;
a variant having amino acid replacements of 49T and 83Y in a sequence of SEQ ID NO: 68;
a variant having amino acid replacements of 49T, 82A, and 83Y in a sequence of SEQ ID NO: 68; or
a variant having amino acid replacements of 49T, 53E, 57E, and 82A in a sequence of SEQ ID NO: 68;
wherein the positions for the amino acid replacements described above are amino acid residue positions numbered in natural order relative to the sequence SEQ ID NO: 8.

In some embodiments, provided is the TACI polypeptide described above, wherein the polypeptide has a sequence set forth in any one of SEQ ID NO: 10, SEQ ID NOs: 13-15, and SEQ ID NOs: 18-35. In some embodiments, provided is the TACI polypeptide described above, wherein the polypeptide has a sequence set forth in any one of SEQ ID NOs: 68-70.

In some embodiments, the TACI polypeptide described above has a sequence set forth in SEQ ID NO: 32, SEQ ID NO: 33, SEQ ID NO: 34, or SEQ ID NO: 35.

In some embodiments, the present disclosure provides a TACI fusion protein, which comprises the TACI polypeptide according to any one of the embodiments described above.

In some embodiments, the present disclosure provides a TACI fusion protein, which comprises the TACI polypeptide according to any one of the embodiments described above (e.g., SEQ ID NO: 32, SEQ ID NO: 33, SEQ ID NO: 34, or SEQ ID NO: 35), and further comprises an immunoglobulin constant region.

In some embodiments, provided is the TACI fusion protein described above, which comprises: any one of the TACI polypeptides described above; and an Fc region. In some embodiments, the Fc region comprises a first subunit and a second subunit, wherein the first subunit and the second subunit of the Fc region may be identical or different.

In some embodiments, provided is the TACI fusion protein described above, wherein the Fc region comprises two subunits capable of associating with each other. In some embodiments, the two subunits are a first subunit and a second subunit, which are identical or different. In some embodiments, the Fc region is an Fc region of an IgG; in some embodiments, the Fc region is an Fc region of human IgG1.

In some embodiments, provided is the TACI fusion protein described above, wherein the Fc region described above comprises one or more amino acid substitutions, wherein the amino acid substitutions are capable of reducing the binding of the Fc region to an Fc receptor as compared to a wild-type Fc region; in some embodiments, the amino acid substitutions described above are capable of reducing binding of the Fc region to an Fcy receptor; in some embodiments, the Fc region has a YTE mutation (M252Y, S254T, and T256E), L234A and L235A mutations, and/or an S228P mutation, wherein the mutation positions are numbered according to the EU index.

In some embodiments, provided is the TACI fusion protein described above, wherein the Fc region described above comprises a first subunit and a second subunit capable of associating with each other, wherein the first subunit and the second subunit have one or more amino acid substitutions for reducing homodimerization. In some embodiments, the first subunit has a protuberance structure according to the knob-into-hole technique and the second subunit has a pore structure according to the knob-into-hole technique, or the first subunit has a pore structure according to the knob-into-hole technique and the second subunit has a protuberance structure according to the knob-into-hole technique. In some embodiments, the amino acid residue substitutions of the first subunit include one or more amino acid substitutions at positions selected from the group consisting of positions 354, 356, 358, 366, 394, 405, and 407, and the amino acid residue substitutions of the second subunit include one or more amino acid substitutions at positions selected from the group consisting of positions 349, 356, 358, 366, 368, 407, 394, and 405. In some embodiments, the amino acid residue substitutions of the first subunit include one or more amino acid substitutions at positions selected from the group consisting of positions 354, 356, 358, and 366, and the amino acid residue substitutions of the second subunit include one or more amino acid substitutions at positions selected from the group consisting of positions 349, 356, 358, 366, 368, and 407. In some embodiments, the first subunit comprises one or more amino acid substitutions selected from the group consisting of 354C, 356E, 358M, and 366W, and the second subunit comprises one or more amino acid substitutions selected from the group consisting of 349C, 356E, 358M, 366S, 368A, and 407V In some embodiments, the first subunit comprises amino acid substitutions of 354C, 356E, 358M, and 366W, and the second subunit comprises amino acid substitutions of 349C, 356E, 358M, 366S, 368A, and 407V

In some embodiments, provided is the TACI fusion protein described above, wherein the Fc region described above has a sequence set forth in SEQ ID NO: 11, SEQ ID NO: 61, SEQ ID NO: 62, or SEQ ID NO: 63, or a sequence having at least 90%, 95%, 96%, 97%, 98%, or 99% sequence identity to SEQ ID NO: 11, SEQ ID NO: 61, SEQ ID NO: 62, or SEQ ID NO: 63. In some embodiments, provided is the TACI fusion protein described above, wherein the first subunit and the second subunit of the Fc region described above are identical and have a sequence set forth in SEQ ID NO: 11, SEQ ID NO: 61, SEQ ID NO: 62, or SEQ ID NO: 63.

In some embodiments, provided is the TACI fusion protein according to any one of the embodiments described above, wherein each of the TACI fusion protein molecules comprises 1, 2, 3, or 4 BAFF-binding domains, and an Fc region. In some embodiments, provided is the TACI fusion protein, wherein each of the TACI fusion protein molecules comprises 2 TACI polypeptides and an Fc region. In some embodiments, each of the TACI fusion protein molecules comprises 4 TACI polypeptides and an Fc region.

In some embodiments, provided is the TACI fusion protein according to any one of the embodiments described above, wherein the TACI polypeptide is linked to one subunit of the Fc region in any order. In some embodiments, the TACI polypeptide is linked to the first subunit or the second subunit of the Fc region via a linker or is directly linked to the first subunit or the second subunit of the Fc region. In some embodiments, the C-terminus of the TACI polypeptide is linked to the N-terminus of the first subunit or the second subunit of the Fc region via a linker or directly; or the N-terminus of the TACI polypeptide is linked to the C-terminus of the first subunit or the second subunit of the Fc region via a linker or directly.

In some embodiments, provided is the TACI fusion protein described above, which is a dimeric protein and comprises a polypeptide chain selected from the group consisting of polypeptide chains shown in a, b, and c:
a. from the N-terminus to the C-terminus: [TACI polypeptide]-[linker]-[subunit of Fc region];
b. from the N-terminus to the C-terminus: [subunit of Fc region]-[linker]-[TACI polypeptide]; and
c. from the N-terminus to the C-terminus: [TACI polypeptide 1]-[linker 1]-[subunit of an Fc region]-[linker 2]-[TACI polypeptide 2], wherein linker 1 and linker 2 may be identical or different, and TACI polypeptide 1 and TACI polypeptide 2 may be identical or different; the subunit of the Fc region is a first subunit or a second subunit of the Fc region.

In some embodiments, provided is the TACI fusion protein described above, wherein the linker is a linker set forth in SEQ ID NO: 64, or the linker is selected from the group consisting of a (GₓS)_{y} linker, wherein X is an integer selected from the group consisting of 1 to 5, and Y is an integer selected from the group consisting of 0 to 6. When Y is 0, it means that the linker is a bond, and the TACI polypeptide is directly linked to the subunit of the Fc region via the bond. In some embodiments, provided is the (GₓS)_{y} linker, wherein X is an integer from 1 to 5 (e.g., X is 4) and Y is an integer selected from the group consisting of 1 to 6 (e.g., 1, 2, 3, 4, 5, or 6). In some embodiments, the linker is a linker set forth in SEQ ID NO: 65 or SEQ ID NO: 66.

In some embodiments, provided is the TACI fusion protein described above, which comprises 2 identical polypeptide chains. In some embodiments, the TACI fusion protein described above comprises 2 identical polypeptides set forth in any one of SEQ ID NOs: 36-44, or a polypeptide chain comprising an amino acid sequence having at least 90%, 95%, 96%, 97%, 98%, or 99% sequence identity to any one of SEQ ID NOs: 36-44.

In some embodiments, provided is the TACI fusion protein described above, which is a fusion protein of TACI and an antibody and comprises a TACI polypeptide and an antibody (hereinafter also referred to as a TACI antibody fusion protein), wherein the TACI polypeptide is the TACI polypeptide according to any one of the embodiments described above, and the antibody is an anti-IL23 antibody, an anti-IL12 antibody, or an anti-IL23/IL12 p40 subunit antibody.

In some embodiments, provided is the TACI antibody fusion protein described above, which comprises a TACI polypeptide and an anti-IL23/IL12 p40 subunit antibody (the p40 subunit is a subunit shared by IL23 and IL12).

In some embodiments, provided is the TACI antibody fusion protein described above, which comprises the TACI polypeptide according to any one of the embodiments described above, and an antibody. In some embodiments, the antibody is an anti-IL23 antibody, an anti-IL12 antibody, or an anti-IL23/IL12 p40 subunit antibody.

In some embodiments, provided is the TACI antibody fusion protein described above, wherein the antibody comprises a heavy chain variable region and a light chain variable region, wherein the heavy chain variable region comprises a HCDR1, a HCDR2, and a HCDR3 set forth in SEQ ID NO: 52, SEQ ID NO: 53, and SEQ ID NO: 54, respectively, and the light chain variable region comprises a LCDR1, a LCDR2, and a LCDR3 set forth in SEQ ID NO: 49, SEQ ID NO: 50, and SEQ ID NO: 51, respectively. The CDRs are defined according to Kabat numbering scheme.

In some embodiments, provided is the TACI antibody fusion protein described above, wherein the antibody comprises a heavy chain variable region set forth in SEQ ID NO: 45 and a light chain variable region set forth in SEQ ID NO: 46.

In some embodiments, provided the TACI antibody fusion protein described above, wherein the antibody comprises an antibody heavy chain constant region and an antibody light chain constant region. In some embodiments, the heavy chain constant region is selected from the group consisting of human IgG1, IgG2, IgG3, and IgG4 constant regions, and the light chain constant region is selected from the group consisting of human antibody κ and λ chain constant regions. In some embodiments, provided is the TACI antibody fusion protein, wherein the antibody heavy chain constant region comprises one or more amino acid substitutions, wherein the amino acid substitutions are capable of reducing the binding of the heavy chain constant region to an Fc receptor. In some embodiments, the amino acid substitutions described above are capable of reducing the binding of the heavy chain constant region to an Fcγ receptor. In some embodiments, the Fc region has a YTE mutation (M252Y, S254T, and T256E), L234A and L235A mutations, and/or an S228P mutation, wherein the mutations are numbered according to the EU index. In some embodiments, the antibody heavy chain constant region described above comprises a first subunit and a second subunit capable of associating with each other, wherein the first subunit and the second subunit have one or more amino acid substitutions for reducing homodimerization. In some embodiments, the first subunit has a protuberance structure according to the knob-into-hole technique and the second subunit has a pore structure according to the knob-into-hole technique, or the first subunit has a pore structure according to the knob-into-hole technique and the second subunit has a protuberance structure according to the knob-into-hole technique. In some embodiments, the amino acid residue substitutions of the first subunit include one or more amino acid substitutions at positions selected from the group consisting of positions 354, 356, 358, and 366, and the amino acid residue substitutions of the second subunit include one or more amino acid substitutions at positions selected from the group consisting of positions 349, 356, 358, 366, 368, and 407. In some embodiments, the first subunit comprises one or more amino acid substitutions selected from the group consisting of 354C, 356E, 358M, and 366W, and the second subunit comprises one or more amino acid substitutions selected from the group consisting of 349C, 356E, 358M, 366S, 368A, and 407V In some embodiments, the first subunit comprises amino acid substitutions of 354C, 356E, 358M, and 366W, and the second subunit comprises amino acid substitutions of 349C, 356E, 358M, 366S, 368A, and 407V

In some embodiments, provided is the TACI antibody fusion protein described above, wherein the antibody comprises a heavy chain having at least 85% sequence identity to SEQ ID NO: 47 and a light chain having at least 85% sequence identity to SEQ ID NO: 48; in some embodiments, the antibody comprises a heavy chain set forth in SEQ ID NO: 47 and a light chain set forth in SEQ ID NO: 48.

In some embodiments, provided is the TACI antibody fusion protein described above, wherein the TACI polypeptide is linked to an antibody heavy chain or light chain directly or via a linker. In some embodiments, the C-terminus of the TACI polypeptide is linked to the N-terminus of the antibody heavy or light chain via a linker; in some embodiments, the N-terminus of the TACI polypeptide is linked to the C-terminus of the antibody heavy or light chain via a linker; in some embodiments, the C-terminus of the TACI polypeptide is directly linked to the N-terminus of the antibody heavy or light chain; in some embodiments, the N-terminus of the TACI polypeptide is directly linked to the C-terminus of the antibody heavy or light chain.

In some embodiments, provided is the TACI antibody fusion protein described above, wherein each of the TACI antibody fusion protein molecules comprises 1, 2, 3, 4, or more TACI polypeptides. In some embodiments, provided is the TACI antibody fusion protein, wherein each of the TACI antibody fusion protein molecules comprises 1 TACI polypeptide. In some embodiments, provided is the TACI antibody fusion protein, wherein each of the TACI antibody fusion protein molecules comprises 2 TACI polypeptides. In some embodiments, provided is the TACI antibody fusion protein, wherein each of the TACI antibody fusion protein molecules comprises 3 TACI polypeptides. In some embodiments, provided is the TACI antibody fusion protein, wherein each of the TACI antibody fusion protein molecules comprises 4 TACI polypeptides.

In some embodiments, provided is the TACI antibody fusion protein, wherein for the TACI polypeptide, the N-terminus of at least one of the TACI polypeptides is linked to the C-terminus of the antibody heavy chain. In some embodiments, provided is the TACI antibody fusion protein, wherein the N-terminus of at least one of the TACI polypeptides is linked to the C-terminus of the antibody light chain. In some embodiments, provided is the TACI antibody fusion protein, wherein the C-terminus of at least one of the TACI polypeptides is linked to the N-terminus of the antibody heavy chain. In some embodiments, provided is the TACI antibody fusion protein, wherein the C-terminus of at least one of the TACI polypeptides is linked to the N-terminus of the antibody light chain.

In some embodiments, provided is the TACI antibody fusion protein described above, which comprises a polypeptide chain as described in d or e below:
d. a first polypeptide chain comprising [antibody heavy chain]-[linker]-[TACI polypeptide] from the N-terminus to the C-terminus, and
a second polypeptide chain being an antibody light chain; wherein the antibody heavy and light chains form an antigen-binding site;
e. a first polypeptide chain comprising [antibody heavy chain]-[linker]-[TACI polypeptide] from the N-terminus to the C-terminus, and
a second polypeptide chain comprising [TACI polypeptide]-[linker]-[antibody light chain], wherein the linker may or may not be present; the antibody heavy and light chains form an antigen-binding site.

In some embodiments, provided is the TACI antibody fusion protein described above, wherein the linker may be selected from the group consisting of a (GxS)y linker, wherein X is an integer selected from the group consisting of 1 to 5, and Y is an integer selected from the group consisting of 0 to 6. When Y is 0, it means that the linker is a bond, and the TACI polypeptide is directly linked to the subunit of the Fc region via the bond. In some embodiments, X is an integer from 1 to 5 (e.g., X is 4), and Y is an integer selected from the group consisting of 1 to 6 (e.g., 1, 2, 3, 4, 5, or 6). In some embodiments, the linker is a linker set forth in SEQ ID NO: 65, SEQ ID NO: 66, or SEQ ID NO: 67. In some embodiments, the antibody is a murine antibody, a chimeric antibody, a humanized antibody, or a fully human antibody.

In some embodiments, provided is the TACI antibody fusion protein described above, which comprises two identical first polypeptide chains set forth in SEQ ID NO: 55 and two identical second polypeptide chains set forth in SEQ ID NO: 56. In some embodiments, provided is the TACI antibody fusion protein described above, which comprises two identical first polypeptide chains set forth in SEQ ID NO: 57 and two identical second polypeptide chains set forth in SEQ ID NO: 58.

In some embodiments, the present disclosure provides a pharmaceutical composition comprising: the TACI polypeptide or the TACI fusion protein described above, and one or more pharmaceutically acceptable carriers, diluents, or excipients.

In some embodiments, the present disclosure provides an isolated nucleic acid molecule (polynucleotide) encoding the TACI polypeptide or the TACI fusion protein according to any one of the embodiments described above.

In some embodiments, the present disclosure provides an expression vector comprising the nucleic acid molecule described above (DNA or RNA).

In some embodiments, the present disclosure provides a host cell comprising the nucleic acid molecule described above.

In some embodiments, the present disclosure provides a host cell comprising the expression vector described above.

The host cell provided by the present disclosure cannot develop into an animal or plant individual.

In some embodiments, the present disclosure provides a method for treating or ameliorating a B cell disorder or an autoimmune disease, which comprises the step of administering to a subject in need thereof a therapeutically effective amount of the TACI polypeptide, the TACI fusion protein, or the pharmaceutical composition according to any one of the embodiments described above. In some embodiments, the B cell disorder or the autoimmune disease is a disease or condition associated with TACI expression. In some embodiments, the autoimmune disease is selected from the group consisting of: systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of: tumors, chronic leukocytic leukemia, multiple myeloma, non-Hodgkin's lymphoma, post-transplant lymphoproliferative disorder, and light chain gammopathy. In some embodiments, the autoimmune disease is systemic lupus erythematosus.

In some embodiments, the present disclosure provides use of the TACI polypeptide, the TACI fusion protein, the nucleic acid molecule, or the pharmaceutical composition according to any one of the embodiments described above in the preparation of a medicament for treating or preventing a disease. The present disclosure also provides the TACI polypeptide, the TACI fusion protein, the nucleic acid molecule, or the pharmaceutical composition according to any one of the embodiments described above, for use in treating or preventing a disease. In some embodiments, the disease is a B cell disorder or an autoimmune disease. In some embodiments, the B cell disorder or the autoimmune disease is a disease or condition associated with TACI expression. In some embodiments, the autoimmune disease is selected from the group consisting of: systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of: tumors, chronic leukocytic leukemia, multiple myeloma, non-Hodgkin's lymphoma, post-transplant lymphoproliferative disorder, and light chain gammopathy. In some embodiments, the autoimmune disease is systemic lupus erythematosus.

In another aspect, the present disclosure also provides the TACI polypeptide, the TACI fusion protein, the nucleic acid molecule, or the composition according to any one of the embodiments described above, for use as a medicament. In some embodiments, the medicament is used for treating a B cell disorder or an autoimmune disease. In some embodiments, the B cell disorder or the autoimmune disease is a disease or condition associated with TACI expression. In some embodiments, the autoimmune disease is selected from the group consisting of: systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of: tumors, chronic leukocytic leukemia, multiple myeloma, non-Hodgkin's lymphoma, post-transplant lymphoproliferative disorder, and light chain gammopathy. In some embodiments, the autoimmune disease is systemic lupus erythematosus.

### BRIEF DESCRIPTION OF THE DRAWINGS

FIG. 1: a schematic structural diagram of a TACI fusion protein (N-terminal second-order TACI fusion protein of Fc, i.e., a fusion protein comprising two TACI polypeptides, wherein the N-terminus of each of the first subunit and the second subunit of the Fc region is linked to one TACI polypeptide).
FIG. 2: a schematic structural diagram of a TACI fusion protein (fourth-order TACI fusion protein of Fc, i.e., a fusion protein comprising 4 TACI polypeptides, wherein the N-terminus and the C-terminus of each of the first subunit and the second subunit of the Fc region is linked to one TACI polypeptide).
FIG. 3: a schematic structural diagram of a TACI fusion protein (C-terminal second-order TACI fusion protein of Fc, i.e., a fusion protein comprising two TACI polypeptides, wherein the C-terminus of each of the first subunit and the second subunit of the Fc region is linked to one TACI polypeptide).
FIG. 4: a schematic structural diagram of a TACI antibody fusion protein (C-terminal second-order TACI fusion protein of antibody heavy chains, i.e., a fusion protein comprising two TACI polypeptides, wherein the C-terminus of each of the two heavy chains is linked to one TACI polypeptide).
FIG. 5: a schematic structural diagram of a TACI antibody fusion protein (antibody light-chain N-terminal and heavy-chain C-terminal fourth-order TACI fusion protein, i.e., a fusion protein comprising 4 TACI polypeptides, wherein the C-terminus of each of the two heavy chains is linked to one TACI polypeptide, and the N-terminus of each of the two light chains is linked to one TACI polypeptide).
FIG. 6: experimental results for the blocking of the binding of BAFF to BAFF-R by TACI antibody fusion proteins.
FIG. 7: experimental results for the inhibition of IFNγ secretion by TACI antibody fusion proteins.
FIG. 8: experimental results for the inhibition of IgA secretion by TACI antibody fusion proteins.
FIG. 9: experimental results for the inhibition of IL-22 secretion by TACI antibody fusion proteins.
FIG. 10: experimental results for the inhibition of TNFα secretion by TACI antibody fusion proteins.
FIG. 11: renal pathological score results for TACI antibody fusion proteins in a pharmacodynamic experiment of SLE models.
FIG. 12: urine protein assay results for TACI antibody fusion proteins in a pharmacodynamic experiment of SLE models.
FIG. 13: dsDNA IgG assay results for TACI antibody fusion proteins in a pharmacodynamic experiment of SLE models.

### DETAILED DESCRIPTION

### Terms

To facilitate the understanding of the present disclosure, some technical and scientific terms are described below. Unless otherwise specifically defined herein, all technical and scientific terms used herein have the same meaning as commonly understood by those of ordinary skill in the art.

As used in the description and in the claims, the singular forms of "a", "an", and "the" include plural referents unless otherwise clearly indicated in the context.

Unless otherwise clearly stated in the context, throughout the description and the claims, the words "comprise", "have", "include", and the like, should be construed in an inclusive sense as opposed to an exclusive or exhaustive sense.

The term "cytokine" is a general term for proteins that are released by a cell population to act on other cells as intercellular mediators. Examples of such cytokines include lymphokines, monokines, chemokines, and traditional polypeptide hormones. Exemplary cytokines include: IL-2, IFN-γ, IL-6, TNFα, IL-17, and IL-5.

TACI described herein is a membrane-bound receptor, and the wild-type human TACI comprises an extracellular region comprising two cysteine-rich pseudo-repeats, a transmembrane region, and a cytoplasmic region that interacts with CAML (calcium-modulating cyclophilin ligand). The extracellular region (positions 1 to 165) of the wild-type human TACI are set forth in SEQ ID NO: 1.

"TACI extracellular domain" and "TACI extracellular region" herein are used interchangeably.

The term "and/or" is intended to include two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used herein are as described in J. *biol. chem,* 243, p3558 (1968).

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a structure different from the general chemical structure of amino acids, but function in a manner similar to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various expressions may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W.

The term "antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. "Native antibody" refers to a naturally-occurring immunoglobulin molecule. For example, a native IgG antibody is a heterotetrameric glycoprotein of about 150,000 Daltons composed of two identical light chains and two identical heavy chains linked by a disulfide bond. From the N-terminus to the C-terminus, each heavy chain has one variable region (VH), also known as variable heavy domain or heavy chain variable region, followed by heavy chain constant regions. Generally, a native IgG heavy chain constant region comprises three constant domains (CH1, CH2, and CH3). Similarly, from the N-terminus to the C-terminus, each light chain has one variable region (VL), also known as variable light domain or light chain variable domain, followed by one constant light domain (light chain constant region, CL). The term "full-length antibody", "intact antibody", and "whole antibody" are used herein interchangeably and refer to an antibody having a substantially similar structure to a native antibody structure or having heavy chains in an Fc region as defined herein. In a native intact antibody, the light chain comprises light chain variable region VL and constant region CL, the VL being positioned at the amino terminus of the light chain, and the light chain constant region comprises a κ chain and a λ chain; the heavy chain comprises a variable region VH and constant regions (CH1, CH2, and CH3), the VH being positioned at the amino terminus of the heavy chain, and the constant regions being positioned at the carboxyl terminus, wherein CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term antibody "variable region" or "variable domain" refers to a domain in the heavy or light chain of an antibody that is involved in the binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDR regions: HCDR1, HCDR2, and HCDR3; a VL comprises 3 CDR regions: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al. (1991), "Sequences of Proteins of Immunological Interest", 5^{th} ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships among the various numbering schemes are well known to those skilled in the art and are exemplary, as shown in Table 1 below.

**Table 1. Relationships among CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs herein.

The term "antibody fragment" refers to a molecule different from an intact antibody, which comprises a moiety of an intact antibody that binds to an antigen to which the intact antibody binds. Examples of antibody fragments include, but are not limited to, Fv, Fab, Fab', Fab'-SH, F(ab')₂, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native Fc regions and modified Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "chimeric antibody" refers to an antibody in which a portion of the heavy and/or light chain in the antibody is derived from a particular source or species, while the remainder of the heavy and/or light chain is derived from another different source or species.

The term "humanized antibody" is an antibody that retains the reactivity of a non-human antibody while having low immunogenicity in humans. For example, this can be achieved by retaining the non-human CDR regions and replacing the remainder of the antibody with its human counterparts (i.e., the constant regions and the framework region portion of the variable regions).

The terms "human antibody", "humanized antibody", "fully human antibody", and "complete human antibody" are used interchangeably and refer to antibodies in which the variable and constant regions are human sequences. The term encompasses antibodies that are derived from human genes but have, for example, sequences that have been altered to, e.g., reduce possible immunogenicity, increase affinity, eliminate cysteines that may cause undesired folding, or generate glycosylation sites. The term encompasses antibodies recombinantly produced in non-human cells that may confer glycosylation not characteristic of human cells. The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "affinity" refers to the overall strength of a non-covalent interaction between a single binding site of a molecule (e.g., an antibody) and its binding partner (e.g., an antigen). Unless otherwise indicated, as used herein, binding "affinity" refers to an internal binding affinity that reflects a 1:1 interaction between members of a binding pair (e.g., an antibody and an antigen). The affinity of a molecule X for its ligand Y can be generally denoted by the dissociation constant (K_{D}). Affinity can be determined by conventional methods known in the art, including those described herein.

As used herein, the term "k_{assoc}" or "kₐ" refers to the association rate of a particular antibody-antigen interaction, while the term "k_{dis}" or "k_{d}" refers to the dissociation rate of a particular antibody-antigen interaction. The term "K_{D}" refers to the dissociation constant, which is obtained from the ratio of k_{d} to kₐ (i.e., k_{d}/kₐ) and denoted by a molar concentration (M). The K_{D} value of an antibody can be determined using methods well known in the art. For example, surface plasmon resonance is determined using a biosensing system such as a biacore system, or affinity in a solution is determined by solution equilibrium titration (SET).

The term "effector function" refers to biological activities which can be attributed to the Fc region of an antibody (either the native sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody preparations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. In some embodiments, the antibody provided by the present disclosure is a monoclonal antibody.

The term "antigen" refers to a molecule or a portion of a molecule that is capable of being selectively recognized or bound by an antigen-binding protein (including, for example, an antibody). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed by a continuous string of amino acids (linear epitopes) or comprise non-contiguous amino acids (conformational epitopes), e.g., non-contiguous amino acids that are in spatial proximity to each other due to folding of the antigen (i.e., by tertiary folding of the antigen). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, for example, but not limited to, alanine scanning, peptide blotting, peptide cleavage analysis, epitope excision, epitope extraction, chemical modification of the antigen *(*Prot. Sci. 9 (2000) 487-496), and cross-blocking.

The term "capable of specifically binding", "specifically binding", or "binding" means that an antibody is capable of binding to a certain antigen or an epitope thereof with higher affinity than to other antigens or epitopes. Generally, an antibody binds to an antigen or epitope thereof with an equilibrium dissociation constant (K_{D}) of about 1×10⁻⁷ or less (e.g., about 1×10⁻⁸ M or less). In some embodiments, the K_{D} for the binding of an antibody to an antigen is 10% or less (e.g., 1%) of the KD for the binding of the antibody to a non-specific antigen (e.g., BSA or casein). K_{D} may be determined using known methods, for example, by a BIACORE^{®} surface plasmon resonance assay. However, an antibody that specifically binds to an antigen or an epitope thereof may have cross-reactivity to other related antigens, e.g. to corresponding antigens from other species (homologous), such as humans or monkeys, e.g., Macaca fascicularis (cynomolgus, cyno), Pan troglodytes (chimpanzee, chimp), or Callithrix jacchus (commonmarmoset, marmoset).

The term "anti-IL23/IL12 p40 subunit antibody" refers to an antibody that is capable of binding with sufficient affinity to the p40 subunit of IL23 and IL12. In one embodiment, the degree of binding to an unrelated antibody that is not IL23 or IL12 protein is less than about 10% of the binding of the antibody to IL23 and IL12, as measured by a BIACORE^{®} surface plasma resonance assay. In certain embodiments, an antibody that binds to IL23 and IL12 has a dissociation constant (K_{D}) of < about 1 µM, < about 100 nM, < about 10 nM, < about 1 nM, < about 0.1 nM, < about 0.01 nM, or < about 0.001 nM (e.g., 10⁻⁸ M or less, e.g., 10⁻⁸ M to 10⁻¹² M, e.g., 10⁻⁹ M to 10⁻¹⁰ M). In certain embodiments, an anti-IL23/IL12 antibody binds to a conserved epitope in IL23/IL12 from a different species.

The term "linker" refers to a linker unit that links two polypeptide fragments, and generally has some flexibility, such that the use of the linker does not result in the loss of the original function of the protein domain. Herein, the linkers present in the same structure may be identical or different. The linker may be a peptide linker comprising one or more amino acids, typically about 1 to 30, 2 to 24, or 3 to 15 amino acids. The linkers used herein may be identical or different.

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcy receptor (FcyR) expressed on the effector cells. For example, NK cells express FcyRIIIa, while monocytes express FcyRI, FcyRII, and FcyRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis" ("ADCP") refers to a mechanism by which antibody-coated target cells are eliminated by internalization of phagocytic cells such as macrophages or dendritic cells.

The term "complement-dependent cytotoxicity" or "CDC" refers to a mechanism for inducing cell death in which the Fc effector domain of a target-binding antibody binds to and activates a complement component C1q, and C1q then activates the complement cascade, resulting in the death of the target cell. Activation of a complement may also result in the deposition of complement components on the surface of target cells that promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA).

"Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding a polypeptide or a fusion protein refers to one or more nucleic acid molecules encoding the polypeptide or fusion protein, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues. The terms apply to amino acid polymers in which one or more amino acid residues are corresponding naturally occurring amino acids or artificial chemical mimics thereof, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions, when the two sequences are optimally aligned, with gaps introduced as necessary to achieve the maximum percent sequence identity, and without considering any conservative substitutions as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

In the present disclosure, for example, "amino acid residue positions numbered in natural order relative to the sequence SEQ ID NO: XX" refers to positions of a subject sequence corresponding to those of a XX sequence, i.e., relative positions of the two sequences when the subject sequence is optimally aligned with the XX sequence to obtain the highest percent identity. For example, exemplary examples of "amino acid residue positions numbered in natural order relative to SEQ ID NO: 8" in the extracellular region sequences SEQ ID NO: 1 and SEQ ID NO: 10 of TACI are shown in Table 2 below:

**Table 2. Corresponding positions of positions of SEQ ID NO: 8 in other sequences (exemplified by SEQ ID NOs: 1 and 10)**

| Positions in natural order of SEQ ID NO: 8 | Corresponding position | |
|---|---|---|
| | Corresponding positions in SEQ ID NO: 1 | Corresponding positions in SEQ ID NO: 10 |
| Positions 48-88 | Positions 68-108 (SEQ ID NO: 1, in natural order) | Positions 1-41 (SEQ ID NO: 10, in natural order) |
| Positions 48-87 | Positions 68-107 (SEQ ID NO: 1, in natural order) | Positions 1-40 (SEQ ID NO: 10, in natural order) |
| Positions 48-86 | Positions 68-106 (SEQ ID NO: 1, in natural order) | Positions 1-39 (SEQ ID NO: 10, in natural order) |
| Positions 48-85 | Positions 68-105 (SEQ ID NO: 1, in natural order) | Positions 1-38 (SEQ ID NO: 10, in natural order) |
| Position 49 | Position 69 (SEQ ID NO: 1, in natural order) | Position 2 (SEQ ID NO: 10, in natural order) |
| Position 52 | Position 72 (SEQ ID NO: 1, in natural order) | Position 5 (SEQ ID NO: 10, in natural order) |
| Position 53 | Position 73 (SEQ ID NO: 1, in natural order) | Position 6 (SEQ ID NO: 10, in natural order) |
| Position 57 | Position 77 (SEQ ID NO: 1, in natural order) | Position 10 (SEQ ID NO: 10, in natural order) |
| Position 65 | Position 85 (SEQ ID NO: 1, in natural order) | Position 18 (SEQ ID NO: 10, in natural order) |
| Position 82 | Position 102 (SEQ ID NO: 1, in natural order) | Position 35 (SEQ ID NO: 10, in natural order) |
| Position 83 | Position 103 (SEQ ID NO: 1, in natural order) | Position 36 (SEQ ID NO: 10, in natural order) |

| | | |
|---|---|---|
| Note: For example, residue position 49 in natural order of SEQ ID NO: 10 relative to the sequence SEQ ID NO: 8 is residue position 2 (natural order) of SEQ ID NO: 10, and residue position 49 numbered in natural order of SEQ ID NO: 1 relative to the sequence SEQ ID NO: 8 is position 69 (natural order) of SEQ ID NO: 1. | | |

The term "fusion" or "linkage" means that components (e.g., TACI polypeptide and Fc domain) are linked by a covalent bond, either directly or via one or more linkers. When the linker is a peptide linker, the covalent bond is a peptide bond.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progenies derived therefrom, regardless of the number of passages. Progeny may not be exactly the same as parent cells in terms of nucleic acid content and may contain mutations. Mutant progenies that have the same function or biological activity as the cells screened or selected from the initially transformed cells are included herein. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae, Pichia thermotolerans, Pichia salictaria, Pichia guercuum, Pichia pijperi, Pichia stiptis, Pichia stiptis, Pichia methanolica, Pichia, Saccharomycescerevisiae, Saccharomyces, Hansenula polymorpha, Kluyveromyces, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens, Neurospora crassa, Aspergillus,* and *Yarrowia lipolytica.*

"Optional" or "optionally" means that the event or circumstance subsequently described may, but not necessarily, occur, and that the description includes instances where the event or circumstance occurs or does not occur.

The term "pharmaceutical composition" refers to a mixture comprising one or more of the polypeptides or TACI fusion proteins described herein and other chemical components, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes both human and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to *Macaca fascicularis.* In certain embodiments, the individual or subject is a human.

"Administrating" or "giving", when applied to animals, humans, experimental subjects, cells, tissue, organs, or biological fluids, refer to contact of an exogenous drug, a therapeutic agent, a diagnostic agent, or a composition with the animals, humans, subjects, cells, tissue, organs, or biological fluids.

The term "sample" refers to a collection of fluids, cells, or tissue isolated from a subject, as well as fluids, cells, or tissue present in a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura; pericardium; peritoneum; abdominal cavity and other body cavities; fluids collected from bronchial lavage; synovial fluids; liquid solutions in contact with a subject or biological source, e.g., cell and organ culture media (including cell or organ conditioned media); lavage fluids; and the like), tissue biopsy samples, fine needle punctures, surgically excised tissues, organ cultures, or cell cultures.

"Treatment" or "treat" (and grammatical variations thereof) refer to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the polypeptide or fusion protein of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage caused by or associated with a disease state. In some embodiments, the effective amount is a therapeutically effective amount or a prophylactically effective amount. "Therapeutically effective amount" is an amount sufficient to treat a disease state or condition, particularly a state or condition associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a patient.

### TACI Polypeptide and Fusion Protein Thereof of the Present Disclosure

In one aspect, in the present disclosure, TACI sequence fragments of different lengths are designed, and a novel TACI sequence fragment SEQ ID NO: 8 (which does not comprise amino acid residues at positions 13 and 14 of the extracellular region (positions 13 and 14 in natural order of SEQ ID NO: 1) that cause TACI cleavage) is finally obtained, which has better TACI cleavage resistance and good TACI polypeptide functional activity. In addition, by further truncating the N-terminus and the C-terminus of SEQ ID NO: 8, a TACI polypeptide fragment with better functional activity comprising amino acid residues at positions 48-85 of SEQ ID NO: 8 but not amino acid residues at positions 34-66 of SEQ ID NO: 1 (natural order) is obtained. In addition, amino acid residues at part of the positions of the TACI polypeptide fragment are replaced to obtain a TACI polypeptide with better binding activity, blocking function, stability, and druggability.

### A. Exemplary TACI polypeptides and fusion proteins thereof

In one aspect, the present disclosure provides a TACI polypeptide, which is SEQ ID NO: 8 or a variant thereof, a truncated fragment comprising amino acid residues at positions 48-85 of SEQ ID NO: 8 or a variant thereof, or a truncated fragment comprising amino acid residues at positions 51-85 of SEQ ID NO: 8 or a variant thereof. In particular, a fusion protein comprising the TACI polypeptide of the present disclosure (e.g., TACI-Fc) has one or more of the following functional activities:
a. being not prone to producing fragments caused by TACI cleavage, wherein in some embodiments, the cleavage can be detected by mass spectrometry;
b. the ability to block the binding of BAFF to BAFF-R, wherein in some embodiments, the TACI-Fc blocks the binding of BAFF to BAFF-R with an IC₅₀ value of less than 23 nM, less than 10 nM, less than 6 nM, less than 5 nM, less than 4 nM, less than 3 nM, less than 2 nM, less than 1 nM, or less, the IC₅₀ value being determined by the ELISA method; in some embodiments, the test method for the IC₅₀ value is described in Test Example 2;
c. the ability to bind to BAFF, wherein in some embodiments, the TACI-Fc binds to BAFF with an EC₅₀ value of less than 1 nM, 0.5 nM, 0.4 nM, 0.3 nM, 0.2 nM, 0.1 nM, or less, the EC₅₀ value being determined by the ELISA method; in some embodiments, the test method for the EC₅₀ value is described in the ELISA binding assay (coating an antigen protein) of Test Example 1; in some embodiments, the TACI-Fc binds to BAFF with an EC₅₀ value of less than 30 nM, 20 nM, 10 nM, 9 nM, 8 nM, 7 nM, 6 nM, or less, the EC₅₀ value being determined by the ELISA method (coating a test sample); in some embodiments, the test method for EC₅₀ value is described in the ELISA binding assay (coating a test sample) of Test Example 1;
d. inhibiting BAFF-induced B cell proliferation, wherein in some embodiments, the TACI-Fc inhibits the BAFF-induced B-cell proliferation with an IC₅₀ value of less than 0.3 nM, less than 0.2 nM, less than 0.1 nM, 0.06 nM, less than 0.05 nM, less than 0.01 nM, or less, the IC₅₀ value being determined by the ELISA method; in some embodiments, the test method for the IC₅₀ value is described in Test Example 3;
e. inhibiting APRIL-induced B cell proliferation, wherein in some embodiments, the TACI-Fc inhibits the APRIL-induced B-cell proliferation with an IC₅₀ value of less than 3 nM, less than 2 nM, less than 1 nM, less than 0.5 nM, less than 0.4 nM, less than 0.3 nM, 0.2 nM, less than 0.1 nM, less than 0.05 nM, or less, the IC₅₀ value being determined by the ELISA method; in some embodiments, the test method for the IC₅₀ value is described in Test Example 3;
f. binding with high affinity to human BAFF and/or human APRII,, wherein in some embodiments, the affinity is determined by the Biacore method; in some embodiments, the affinity is determined by the method of Test Example 5; in some embodiments, the TACI-Fc fusion protein binds to human BAFF with a K_{D} value of less than 1E-10 M, less than 9E-11 M, less than 8E-11 M, or less; in some embodiments, the TACI-Fc fusion protein binds to human APRII, with a K_{D} value of less than 2.3E-11 M, less than 2.0E-11 M, 1.9E-11 M, 1.3E-11 M, or less;
g. good *in vivo* pharmacokinetics, wherein in some embodiments, the TACI-Fc has an *in vivo* half-life of more than 4 days in rats; in some embodiments, the test method for the half-life is described in Test Example 7; and/or
h. better stability, wherein in some embodiments, the TACI-Fc fusion protein remains a purity of above 94% (SEC%) when the TACI-Fc is stored at a constant temperature of 40 °C for four weeks; in some embodiments, the TACI-Fc has a pI value of less than 9, less than 8, less than 7, less than 6, or less, as determined by the "18cProt/TrEMBL" system analysis.

Illustratively, the TACI polypeptide of the present disclosure has a sequence set forth in any one of SEQ ID NO: 8, SEQ ID NO: 10, SEQ ID NOs: 13-15, SEQ ID NOs: 18-35, and SEQ ID NOs: 68-70, or an amino acid sequence having at least 85%, 90%, 95%, 96%, 97%, 98%, or 99% sequence identity to any one of SEQ ID NO: 8, SEQ ID NO: 10, SEQ ID NOs: 13-15, SEQ ID NOs: 18-35, and SEQ ID NOs: 68-70.

### B. Exemplary TACI antibody fusion protein

In one aspect, the TACI fusion protein of the present disclosure is an antibody fusion protein comprising the TACI polypeptide of the present disclosure and an antibody. In some embodiments, the antibody is an antibody for treating or ameliorating a B cell disorder or an autoimmune disease. In some embodiments, provided is the TACI antibody fusion protein of the present disclosure, wherein the antibody is an anti-IL23 antibody, an anti-IL12 antibody, or an anti-IL23/IL12 p40 subunit antibody. In some embodiments, the TACI antibody fusion protein of the present disclosure has the ability to block the binding of IL23/IL12 to its receptor and also has the ability to block the binding of TACI to its receptor. In particular, a fusion protein comprising the TACI polypeptide of the present disclosure (e.g., a TACI antibody fusion protein) has one or more of the following functional activities:
a. being not prone to producing fragments caused by TACI cleavage, wherein in some embodiments, the cleavage can be detected by mass spectrometry;
b. the ability to block the binding of BAFF to BCMA, wherein in some embodiments, the TACI antibody fusion protein blocks the binding of BAFF to BCMA with an IC₅₀ value of less than 0.4 nM, less than 0.3 nM, less than 0.2 nM, less than 0.1 nM, or less, the IC₅₀ value being determined by the ELISA method; in some embodiments, the test method for the IC₅₀ value is described in Test Example 2;
c. the ability to block the binding of BAFF to TACI, wherein in some embodiments, the TACI antibody fusion protein blocks the binding of BAFF to TACI with an IC₅₀ value of less than 0.5 nM, less than 0.4 nM, less than 0.3 nM, less than 0.2 nM, less than 0.1 nM, or less, the IC₅₀ value being determined by the ELISA method; in some embodiments, the test method for the IC₅₀ value is described in Test Example 2;
d. the ability to block the binding of APRIL to BCMA, wherein in some embodiments, the TACI antibody fusion protein blocks the binding of APRIL to BCMA with an IC₅₀ value of less than 25 nM, less than 20 nM, less than 15 nM, less than 10 nM, less than 5 nM, less than 4 nM, less than 3 nM, less than 2 nM, less than 1 nM, or less, the IC₅₀ value being determined by the ELISA method; in some embodiments, the test method for the IC₅₀ value is described in Test Example 2;
e. the ability to block the binding of APRIL to TACI, wherein in some embodiments, the TACI antibody fusion protein blocks the binding of APRIL to TACI with an IC₅₀ value of less than 3 nM, less than 2 nM, less than 1 nM, less than 0.9 nM, less than 0.5 nM, or less, the IC₅₀ value being determined by the ELISA method; in some embodiments, the test method for the IC₅₀ value is described in Test Example 2;
f. the ability to block the binding of p40 to IL-12Rβ1, wherein in some embodiments, the TACI antibody fusion protein blocks the binding of p40 to IL-12Rβ1 with an IC₅₀ value of less than 3 nM, less than 2 nM, less than 1 nM, less than 0.9 nM, less than 0.5 nM, or less, the IC₅₀ value being determined by the ELISA method; in some embodiments, the test method for the IC₅₀ value is described in Test Example 2;
g. the ability to block the binding of BAFF to BAFF-R, wherein in some embodiments, the IC₅₀ value for blocking the binding of BAFF to BAFF-R is determined by the ELISA method; in some embodiments, the test method for the IC₅₀ value is described in Test Example 2;
h. the ability to bind to BAFF, wherein in some embodiments, the TACI antibody fusion protein binds to BAFF with an EC₅₀ value of less than 6 nM, 5 nM, 4 nM, 3 nM, or less, the EC₅₀ value being determined by the ELISA method; in some embodiments, the test method for the EC₅₀ value is described in the ELISA binding assay (coating an antigen protein) of Test Example 1;
i. the ability to bind to APRIL, wherein in some embodiments, the TACI antibody fusion protein binds to APRIL with an EC₅₀ value of less than 5 nM, 4 nM, 3 nM, 2 nM, 1 nM, or less, the EC₅₀ value being determined by the ELISA method; in some embodiments, the test method for the EC₅₀ value is described in the ELISA binding assay (coating an antigen protein) of Test Example 1;
j. the ability to bind to IL-12/23 p40, wherein in some embodiments, the TACI antibody fusion protein binds to IL-12/23 p40 with an EC₅₀ value of less than 5 nM, 4 nM, 3 nM, 2 nM, 1 nM, 0.7 nM, 0.6 nM, or less, the EC₅₀ value being determined by the ELISA method; in some embodiments, the test method for the EC₅₀ value is described in the ELISA binding assay (coating an antigen protein) of Test Example 1;
k. inhibiting BAFF-induced B cell proliferation, wherein in some embodiments, the TACI antibody fusion protein inhibits the BAFF-induced B-cell proliferation with an IC₅₀ value of less than 0.3 nM, less than 0.2 nM, less than 0.1 nM, less than 0.06 nM, less than 0.05 nM, less than 0.01 nM, or less, the IC₅₀ value being determined by the ELISA method; in some embodiments, the test method for the IC₅₀ value is described in Test Example 3;
l. inhibiting APRIL-induced B cell proliferation, wherein in some embodiments, the TACI antibody fusion protein inhibits the APRIL-induced B-cell proliferation with an IC₅₀ value of less than 1 nM, less than 0.5 nM, less than 0.4 nM, less than 0.3 nM, 0.2 nM, less than 0.1 nM, less than 0.09 nM, or less, the IC₅₀ value being determined by the ELISA method; in some embodiments, the test method for the IC₅₀ value is described in Test Example 3;
m. inhibiting IL-23-induced IL-17 secretion, wherein in some embodiments, the TACI antibody fusion protein inhibits the IL-23-induced IL-17 secretion with an IC₅₀ value of less than 3 nM, less than 2 nM, less than 2.4 nM, less than 2.0 nM, less than 1.7 nM, less than 1 nM, or less; in some embodiments, the test method for the IC₅₀ value is described in Test Example 4;
n. inhibiting IL-12-induced IFNγ secretion, wherein in some embodiments, the TACI antibody fusion protein inhibits the IL-12-induced IFNγ secretion with an IC₅₀ value of less than 10 nM, less than 5 nM, less than 4 nM, less than 3 nM, less than 1 nM, or less; in some embodiments, the test method for the IC₅₀ value is described in Test Example 4;
o. binding with high affinity to human IL-23, human IL-12, human BAFF, human APRIL, cynomolgus monkey p40, cynomolgus monkey BAFF, cynomolgus monkey APRIL, mouse BAFF, and/or mouse APRIL, wherein in some embodiments, the affinity is determined by the Biacore method; in some embodiments, the affinity assay is described in Test Example 5; in some embodiments, the TACI antibody fusion protein binds to human BAFF with a K_{D} value of less than 1E-10 M, less than 9E-11 M, less than 3E-11 M, or less; in some embodiments, the TACI antibody fusion protein binds to mouse BAFF with a K_{D} value of less than 1E-10 M, less than 8E-11 M, less than 4E-11 M, or less; in some embodiments, the TACI antibody fusion protein binds to cynomolgus monkey BAFF with a K_{D} value of less than 4E-10 M, less than 3E-10 M, less than 1E-11 M, or less; in some embodiments, the TACI antibody fusion protein binds to human APRII, with a K_{D} value of less than 2.8E-11 M, less than 2.2E-11 M, less than 1.5E-11 M, or less; in some embodiments, the TACI antibody fusion protein binds to cynomolgus monkey APRIL with a K_{D} value of less than 4.3E-11 M, less than 3.5E-11 M, less than 3.3E-11 M, or less; in some embodiments, the TACI antibody fusion protein binds to mouse APRII, with a K_{D} value of less than 1E-11 M, less than 9E-12 M, less than 8E-11 M, or less; in some embodiments, the TACI antibody fusion protein binds to human IL-23 with a K_{D} value of less than 1E-12 M, less than 9E-11 M, less than 8E-11 M, or less; in some embodiments, the TACI antibody fusion protein binds to cynomolgus monkey p40 with a K_{D} value of less than 3E-10 M, less than 2.7E-10 M, less than 2.6E-10 M, or less; the TACI antibody fusion protein binds to human IL-12 with a K_{D} value of less than 5E-11 M, less than 4E-11 M, less than 3E-11 M, or less;
p. good *in vivo* pharmacokinetics, wherein in some embodiments, the TACI antibody fusion protein has an *in vivo* half-life of more than 9 days in rats; in some embodiments, the test method for the half-life is described in Test Example 7; and/or
q. good plasma stability, wherein in some embodiments, the TACI antibody fusion protein has a final concentration of 0.1 mg/mL in human plasma and has no detectable TACI cleavage at 37 °C after 14 days of incubation.

Illustratively, the TACI antibody fusion protein comprises a first polypeptide chain having at least 85%, 90%, 95%, 96%, 97%, 98%, or 99% sequence identity to SEQ ID NO: 55 and a second polypeptide chain having at least 85%, 90%, 95%, 96%, 97%, 98%, or 99% sequence identity to SEQ ID NO: 56. In some embodiments, the TACI antibody fusion protein comprises a first polypeptide chain having at least 85%, 90%, 95%, 96%, 97%, 98%, or 99% sequence identity to SEQ ID NO: 57 and a second polypeptide chain having at least 85%, 90%, 95%, 96%, 97%, 98%, or 99% sequence identity to SEQ ID NO: 58.

### Modification of Fc region

In one aspect, the Fc region of the TACI fusion protein of the present disclosure (e.g., TACI-Fc or TACI antibody fusion protein) comprises one or more amino acid substitutions, wherein the one or more amino acid substitutions reduce binding of the Fc region to an Fc receptor, e.g., binding of the Fc region to an Fcy receptor, and reduce or eliminate effector functions. A natural IgG Fc region, specifically an IgG₁ Fc region or an IgG₄ Fc region, may cause the fusion protein of the present disclosure to target cells expressing an Fc receptor, rather than cells expressing an antigen. In some embodiments, the engineered Fc region of the present disclosure exhibits reduced binding affinity for an Fc receptor and/or reduced effector functions. In some embodiments, the engineered Fc region shows above 50%, 80%, 90%, or 95% reduction in binding affinity for an Fc receptor as compared to the native Fc region. In some embodiments, the Fc receptor is an Fcy receptor. In some embodiments, the Fc receptor is a human Fcγ receptor, e.g., FcyRI, FcyRIIa, FcyRIIB, or FcyRIIIa. In some embodiments, the engineered Fc region also has reduced binding affinity for a complement (e.g., C1q) as compared to the native Fc region. In some embodiments, the engineered Fc region has no reduced binding affinity for neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the IgG₁ Fc region, amino acid residue substitutions at positions such as positions 238, 265, 269, 270, 297, 327, and 329 can reduce effector functions. In some embodiments, the Fc region is a human IgG₁ Fc region, and the amino acid residues at positions 234 and 235 are A, as numbered according to the EU index. For the IgG₄ Fc region, amino acid residue substitutions at positions such as position 228 can reduce effector functions.

The fusion protein may comprise different antigen-binding domains fused to the two subunits of the Fc region and thus may result in undesired homodimerization. To improve yield and purity, it would be advantageous to introduce modifications that promote heterodimerization in the Fc region of the fusion protein of the present disclosure. In some embodiments, the Fc region of the present disclosure comprises modifications according to the knob-into-hole (KIH) technique, which involves introducing a protuberance structure (knob) at the interface of the first subunit and a pore structure (hole) at the interface of the second subunit, or vice versa. As such, the protuberance structure can be positioned in the pore structure, thereby promoting the formation of heterodimers and inhibiting the production of homodimers. The protuberance structure is constructed by substituting a small amino acid side chain at the interface of the first subunit with a larger side chain (e.g., tyrosine or tryptophan). The pore structure is created at the interface of the second subunit by substituting a large amino acid side chain with a smaller amino acid side chain (e.g., alanine or threonine). The protuberance and pore structures are prepared by altering the nucleic acid encoding the polypeptide, with optional amino acid substitutions shown in Table 3 below:

**Table 3. Combinations of KIH mutations**

| | | | | | | | | | |
|---|---|---|---|---|---|---|---|---|---|
| First subunit | T366Y | T366W | T394W | F405W | T366W | T366Y | T366W | F405W | 354C |
| | | | | | | | | | 356E |
| | | | | | | F405A | F405W | Y407A | 358M |
| | | | | | | | | | 366W |
| Second subunit | Y407T | Y407A | F405A | T394S | T366S | T394W | T394W | T366W | 349C |
| | | | | | | | | | 356E |
| | | | | | L368A | | | | 358M |
| | | | | | Y407V | Y407T | Y407A | T394S | 366S |
| | | | | | | | | | 368A |
| | | | | | | | | | 407V |

In addition to the knob-into-hole technique, other techniques for modifying the CH3 domain of the heavy chain of multispecific antibodies to achieve heterodimerization are also known in the art, e.g., WO 96/27011, WO 98/050431, EP 1870459, WO 2007/110205, WO 007/147901, WO 2009/089004, WO 2010/129304, WO 2011/90754, WO 2011/143545, WO 2012/058768, WO 2013/157954, and WO 013/096291.

The C-terminus of the Fc region may be an intact C-terminus ending with amino acid residues PGK or a shortened C-terminus, e.g., a shortened C-terminus in which one or two C-terminal amino acid residues have been removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without K447 residue and/or G446 + K447 residue removed. In some embodiments, a composition of intact antibodies comprises antibody populations having a mixture of antibodies with and without K447 residues and/or G446 + K447 residues.

### Recombination method

The TACI polypeptide or fusion protein (e.g., TACI antibody fusion protein) can be produced using recombinant methods. For these methods, one or more isolated nucleic acids encoding the polypeptide or fusion protein are provided.

In one embodiment, the present disclosure provides an isolated nucleic acid encoding the polypeptide or fusion protein described above. Such nucleic acids may each independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing a polypeptide or fusion protein, wherein the method comprises culturing a host cell comprising a nucleic acid encoding the polypeptide or fusion protein, as provided above, under conditions suitable for expression, and optionally recovering the antibody from the host cell (or a host cell medium).

For recombinant production of the polypeptide or fusion protein, the nucleic acid encoding the protein is isolated and inserted into one or more vectors for further cloning and/or expression in a host cell. Such nucleic acids can be readily isolated and sequenced using conventional procedures, or produced by recombinant methods or obtained by chemical synthesis.

Suitable host cells for cloning or expressing a vector encoding the polypeptide or fusion protein include prokaryotic or eukaryotic cells described herein. For example, the polypeptide or fusion protein can be produced in bacteria, particularly when glycosylation and Fc effector functions are not required. After expression, the polypeptide or fusion protein can be isolated from the bacterial cell paste in a soluble fraction and can be further purified.

In addition to prokaryotes, eukaryotic microorganisms such as filamentous fungi or yeast are suitable cloning or expression hosts for vectors encoding fusion proteins, including fungal and yeast strains. Suitable host cells for expression of the fusion protein may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US 5959177, US 6040498, US 6420548, US 7125978, and US 6417429; vertebrate cells can also be used as hosts, e.g., mammalian cell lines adapted for growth in a suspension. Other examples of suitable mammalian host cell lines include a SV40-transformed monkey kidney CV1 line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRCS cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the polypeptide or fusion protein, see, e.g., Yazaki, P. and Wu, A.M., Methods in Molecular Biology, Vol. 248, Lo, B.K.C. (eds.), Humana Press, Totowa, NJ (2004), pp. 255-268.

### Assay

The polypeptide or fusion protein provided herein can be identified, screened, or characterized for their physical/chemical characteristics and/or biological activities by a variety of assays known in the art. In one aspect, the polypeptide or fusion protein of the present disclosure is tested for activity, e.g., by known methods such as ELISA, western blot, and the like.

### Treatment method and route of administration

Any of the TACI polypeptides or fusion proteins thereof (e.g., antibody fusion proteins) provided herein can be used in the treatment method. In yet another aspect, the present disclosure provides use of the TACI polypeptide or the fusion protein thereof in the manufacture or preparation of a medicament. In some embodiments, the B cell disorder or the autoimmune disease is a disease or condition associated with TACI expression. In some embodiments, the autoimmune disease is selected from the group consisting of: systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of: tumors, chronic leukocytic leukemia, multiple myeloma, non-Hodgkin's lymphoma, post-transplant lymphoproliferative disorder, and light chain gammopathy. In some embodiments, the autoimmune disease is systemic lupus erythematosus. In one such embodiment, the use further comprises administering to a subject an effective amount of at least one additional therapeutic agent (e.g., one, two, three, four, five, or six additional therapeutic agents). The "subject" according to any of the above embodiments may be a human.

In yet another aspect, provided is a pharmaceutical composition comprising the polypeptide or fusion protein, e.g., for any of the above pharmaceutical uses or treatment methods. In one embodiment, the pharmaceutical composition comprises any of the polypeptides or fusion proteins provided herein and a pharmaceutically acceptable carrier. In another embodiment, the pharmaceutical composition further comprises at least one additional therapeutic agent.

The polypeptides or fusion proteins of the present disclosure can be used alone or in combination with additional agents for the treatment. For example, the antibody of the present disclosure can be co-administered with at least one additional therapeutic agent. The polypeptides or fusion proteins of the present disclosure (and any additional therapeutic agents) may be administered by any suitable means, including parenteral, intrapulmonary, and intranasal administration, and if required by local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The polypeptide or fusion protein of the present disclosure will be formulated, administered, and applied in a manner consistent with Good Medical Practice (GMP). Factors considered in this context include the specific condition being treated, the specific mammal being treated, the clinical state of the individual patient, the cause of the condition, the site of delivery of the agent, the method of administration, the timing of administration, and other factors known to medical practitioners. The polypeptide or fusion protein may be formulated with or without one or more agents currently used to prevent or treat the condition. The effective amount of such additional agents depends on the amount present in the pharmaceutical composition, the type of condition or treatment, and other factors. These are generally used in the same dosages and routes of administration as described herein, or in about 1% to 99% of the dosages described herein, or in other dosages, and by any route empirically/clinically determined to be appropriate.

For the prevention or treatment of a disease, the appropriate dosage of the polypeptide or fusion protein of the present disclosure (when used alone or in combination with one or more additional therapeutic agents) will depend on the type of disease to be treated, the type of therapeutic molecule, the severity and course of the disease, purpose of administration (prophylactic or therapeutic), previous treatment, clinical history and response to the therapeutic molecule of the patient, and the discretion of the attending physician. The therapeutic molecule is suitably administered to a patient in one or a series of treatments.

### Article of manufacture

In another aspect of the present disclosure, provided is an article of manufacture, which comprises materials useful for the treatment, prevention, and/or diagnosis of the above condition. The article of manufacture comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the TACI polypeptide or fusion protein of the present disclosure. The label or package insert indicates that the composition is to be used to treat the selected condition. Further, the article of manufacture may comprise: (a) a first container containing a composition, wherein the composition comprises the TACI polypeptide or fusion protein of the present disclosure; and (b) a second container containing a composition, wherein the composition comprises other cytotoxic agents or additional therapeutic agents. The article of manufacture in this embodiment of the present disclosure may further comprise a package insert indicating that the composition may be used to treat a particular condition. Alternatively or additionally, the article of manufacture may further comprise a second (or third) container containing a pharmaceutically acceptable buffer. From a commercial and user standpoint, it may further contain other materials as required, including other buffers, diluents, filters, needles, and syringes.

### Examples and Test Examples

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

### Example 1: TACI sequence analysis and engineering

Due to the presence of multiple protease digestion sites in the sequence of TACI, full-length TACI is very susceptible to cleavage after expression. In the experiment, TACI sequence segments of different lengths were designed, and amino acid mutations were introduced at easy-to-cleavage positions of TACI to make the sequences more stable. Exemplary TACI sequences are shown as follows:
> TACI-0 amino acid residues at positions 1-165 of wild-type human TACI) 
MSGLGRSRRGGRSRVDQEERFPQGLWTGVAMRSCPEEQYWDPLLGTCMSCKTI
CNHQSQRTCAAFCRSLSCRKEQGKFYDHLLRDCISCASICGQHPKQCAYFCENK
LRSPVNLPPELRRQRSGEVENNSDNSGRYQGLEHRGSEASPALPGLKLSADQVA
LVYS
SEQ ID NO: 1
> TACI-1 
SGLGRSRKGGKSRVDQEERFPQGLWTGVAMRSCPEEQYWDPLLGTCMSCKTIC
NHQSQRTCAAFCRSLSCRKEQGKFYDHLLRDCISCASICGQHPKQCAYFCENKL
RSPVNLPPELRKQRSGEVEQNSDNSGKYQGLEHRGSEASPALPGLKLSADQVAL
VYS
SEQ ID NO: 2
> TACI-2 
SGLGRSRKGGKSRVDQEERFPQGLWTGVAMRSCPEEQYWDPLLGTCMSCKTIC
NHQSQRTCAAFCRSLSCRKEQGKFYDHLLRDCISCASICGQHPKQCAYFCENKL
RSPVNLPPELRKQRSGEVENNSDNSG
SEQ ID NO: 3
> TACI-3 
SGLGRSRKGGKSRVDQEERFPQGLWTGVAMRSCPEEQYWDPLLGTCMSCKTIC
NHQSQRTCAAFCRSLSCRKEQGKFYDHLLRDCISCASICGQHPKQCAYFCENKL
RSPVNLPPELRKQRSGEVE
SEQ ID NO: 4
> TACI-4 
GGRSRVDQEERFPQGLWTGVAMRSCPEEQYWDPLLGTCMSCKTICNHQSQRTC
AAFCRSLSCRKEQGKFYDHLLRDCISCASICGQHPKQCAYFCENKLRSPVNLPPE
LRKQRSGEVENNSDNSGKYQGLEHRGSEASPALPGLKLSADQVALVYS
SEQ ID NO: 5
> TACI-5 
GGRSRVDQEERFPQGLWTGVAMRSCPEEQYWDPLLGTCMSCKTICNHQSQRTC
AAFCRSLSCRKEQGKFYDHLLRDCISCASICGQHPKQCAYFCENKLRSPVNLPPE
LRKQRSGEVENNSDNSG
SEQ ID NO: 6
> TACI-6 
GGRSRVDQEERFPQGLWTGVAMRSCPEEQYWDPLLGTCMSCKTICNHQSQRTC
AAFCRSLSCRKEQGKFYDHLLRDCISCASICGQHPKQCAYFCENKLRSPVNLPPE
LRKQRSGEVE
SEQ ID NO: 7
> TACI-7 
FPQGLWTGVAMRSCPEEQYWDPLLGTCMSCKTICNHQSQRTCAAFCRSLSCRK
EQGKFYDHLLRDCISCASICGQHPKQCAYFCENKLRSPVNLPP
SEQ ID NO: 8
> TACI-8 
SLSCRKEQGKFYDHLLRDCISCASICGQHPKQCAYFCENKLRSPVNLPPELRKQ
RSGEVENNSDNSGKYQGLEHRGSEASPALPGLKLSADQVALVYS
SEQ ID NO: 9
> TACI-9
   SLSCRKEQGKFYDHLLRDCISCASICGQHPKQCAYFCENKL SEQ ID NO: 10
> Fc sequence of human IgG

DKTHTCPPCPAPEAEGAPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPSSIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNH
YTQKSLSLSPGK
SEQ ID NO: 11

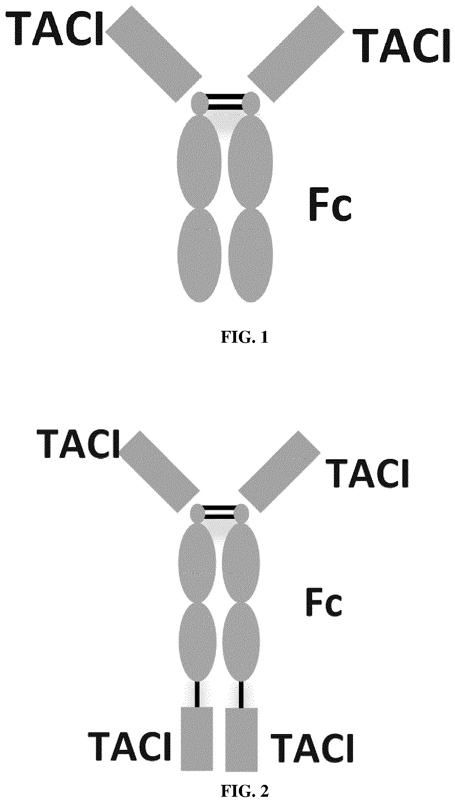

The C-terminus of the above TACI polypeptide fragments was directly fused to the N-terminus of Fc (SEQ ID NO: 11) of human IgG to construct TACI-Fc fusion proteins, which were each transfected and expressed in 293E (human embryonic kidney cell modified by EBNA1 gene), and purified to obtain TACI-Fc fusion proteins comprising two identical polypeptides (see FIG. 1 for the structure). The positive control RCT-18 (Telitacicept) is a TACI-Fc fusion protein constructed from two identical sequences:
> Amino acid sequence of RCT-18

SRVDQEERFPQGLWTGVAMRSCPEEQYWDPLLGTCMSCKTICNHQSQRTCAAF
CRSLSCRKEQGKFYDHLLRDCISCASICGQHPKQCAYFCENKLRSPVNLPPELDK
THTCPPCPAPEAEGAPSVFLFPPKPKDTLMISRTPEVTCVVVDVSHEDPEVKFNW
YVDGVEVHNAKTKPREEQYNSTYRVVSVLTVLHQDWLNGKEYKCKVSNKALP
SSIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQ
KSLSLSPGK
SEQ ID NO: 12

Note: In the sequence of RCT-18, the underlined part indicates a TACI sequence (positions 13-118 of the extracellular region of human TACI), and the non-underlined part indicates an Fc sequence.

The obtained TACI-Fc fusion proteins were analyzed by mass spectrometry. The experimental results are shown in Table 4. The experimental results showed that no TACI cleavage fragment was found in TACI-7Fc or TACI-9Fc, but TACI cleavage fragments were detected in the positive control RCT-18 and other TACI-Fc fusion proteins, e.g., TACI-2 Fc; even though labile amino acids (arginine) at its protease digestion sites (9, 12, 120) had been replaced by more stable amino acids (lysine), TACI cleavage fragments were still detected. The cleavage of RCT-18 was further analyzed (see Test Example 8 of the present application), and TACI cleavage fragments cleaved at positions 1 and 2 of the N-terminus of RCT-18 were found.

**Table 4. Analysis of cleavage of TACI-Fc fusion proteins**

| **Fusion protein** | **TACI moiety** | | | **Analysis results for cleavage by mass spectrometry** |
|---|---|---|---|---|
| | **TACI initiation site** | **TACI ending site** | **Simultaneous introduction of stability mutations** | |
| TACI-1 Fc | 2 | 165 | R9K,R12K,R120K,R135K,N128Q | Cleavage |
| TACI-2 Fc | 2 | 134 | R9K,R12K,R120K | Cleavage |
| TACI-3 Fc | 2 | 127 | R9K,R12K,R120K | Cleavage |
| TACI-4 Fc | 10 | 165 | R120K,R135K | Cleavage |
| TACI-5 Fc | 10 | 134 | R120K | Cleavage |
| TACI-6 Fc | 10 | 127 | R120K | Cleavage |
| TACI-7 Fc | 21 | 116 | No mutation | No cleavage |
| TACI-8 Fc | 68 | 165 | R120K,R135K | Cleavage |
| TACI-9 Fc | 68 | 108 | No mutation | No cleavage |
| RCT-18 | 13 | 118 | No mutation | Cleavage |

| | | | | |
|---|---|---|---|---|
| Note: The positions in the table are residue positions (numbered in natural order) relative to TACI-0 (SEQ ID NO: 1). For example, "R120K" indicates that the amino acid residue at position 120 (numbered in natural order) of the TACI-0 polypeptide is mutated from R to K; the "TACI-5 Fc" fusion protein is a TACI-Fc fusion protein in which the C-terminus of TACI-5 (consisting of amino acid residues at positions 10-134 of TACI-0 after the R120K mutation) is directly fused to the N-terminus of Fc (SEQ ID NO: 11) of human IgG. | | | | |

The above TACI-Fc fusion protein was assayed for the function of blocking the binding of BAFF to BAFF-R (see Test Example 2 for the assay method). The experimental results are shown in Table 5. The experimental results showed that the functional activity of TACI-9 Fc for blocking the binding of BAFF to BAFF-R was significantly stronger than that of the control RCT-18.

**Table 5. Activity of TACI-Fc fusion proteins for blocking the binding of BAFF to BAFF-R**

| TACI fusion protein | IC₅₀ value (nM) of TACI-Fc for blocking the binding of BAFF to BAFF-R |
|---|---|
| TACI-1 Fc | 44.72 |
| TACI-2 Fc | 42.85 |
| TACI-3 Fc | 826.00 |
| TACI-4 Fc | 33.48 |
| TACI-5 Fc | 25.40 |
| TACI-6 Fc | 18.50 |
| TACI-7 Fc | 27.86 |
| TACI-8 Fc | 22.51 |
| TACI-9 Fc | 5.02 |
| RCT-18 | 27.48 |

### Example 2: TACI truncation analysis

The sequence of TACI was further truncated on the basis of TACI-9, and the truncated TACI fragment was assayed for the functional activity. The sequences of the truncated TACI fragments are shown as follows:
> TACI-10 (truncation at the C-terminus of TACI-9 by 1 amino acid "L") SLSCRKEQGKFYDHLLRDCISCASICGQHPKQCAYFCENK
   SEQ ID NO: 13
> TACI-11 (truncation at the C-terminus of TACI-9 by 2 amino acids "KL") SLSCRKEQGKFYDHLLRDCISCASICGQHPKQCAYFCEN
   SEQ ID NO: 14
> TACI-12 (truncation at the C-terminus of TACI-9 by 3 amino acids "NKL") SLSCRKEQGKFYDHLLRDCISCASICGQHPKQCAYFCE
   SEQ ID NO: 15
> TACI-13 (truncation at the C-terminus of TACI-9 by 4 amino acids "ENKL") SLSCRKEQGKFYDHLLRDCISCASICGQHPKQCAYFC
   SEQ ID NO: 16
> TACI-14 (truncation at the C-terminus of TACI-9 by 5 amino acids "CENKL") SLSCRKEQGKFYDHLLRDCISCASICGQHPKQCAYF
   SEQ ID NO: 17

The C-terminus of the above truncated TACI fragments was directly fused to the N-terminus of Fc (SEQ ID NO: 11) of human IgG to construct TACI-Fc fusion proteins, which were each transfected and expressed in 293E (human embryonic kidney cell modified by EBNA1 gene), and purified to obtain TACI-Fc fusion proteins comprising two identical polypeptides (see FIG. 1 for the structure).

The above-constructed TACI-Fc fusion proteins were analyzed by mass spectrometry. The experimental results are shown in Table 6-1. The experimental results showed that none of the TACI-Fc fusion proteins constructed by the truncated TACI-9 fragments had detectable TACI polypeptide cleavage.

In addition, the constructed TACI-Fc fusion proteins were assayed for the activity for binding to BAFF (see the ELISA binding assay (coating an antigen protein) of Test Example 1 of the present disclosure for the experimental method) and for the functional blocking for blocking the binding of BAFF to BAFF-R (see Test Example 2 of the present disclosure for the experimental method). The experimental results are shown in Table 6-1. The experimental results showed that TACI-10 Fc, TACI-11 Fc, and TACI-12 Fc fusion proteins constructed by the truncated TACI-9 fragments TACI-10, TACI-11, and TACI-12 had the same level of activity for binding to BAFF and functional activity for blocking the binding of BAFF to BAFF-R as the TACI-9Fc fusion protein, while the fusion proteins constructed by the truncated fragments TACI-13 and TACI-14 had a significantly weaker function of blocking the binding of BAFF to BAFF-R than the TACI-9Fc fusion protein.

**Table 6-1. Assay results for functional activity and cleavage by mass spectrometry of TACI-Fc fusion proteins**

| Fusion protein | TACI cleavage analysis | EC₅₀ (nM) for the binding of TACI-Fc to BAFF | IC₅₀ (nM) of TACI-Fc for blocking the binding of BAFF to BAFF-R |
|---|---|---|---|
| TACI-9 Fc | No cleavage | 0.4903 | 2.09 |
| TACI-10 Fc | No cleavage | 0.5606 | 2.7 |
| TACI-11 Fc | No cleavage | 0.5841 | 2.621 |
| TACI-12 Fc | No cleavage | 0.8944 | 3.862 |
| TACI-13 Fc | No cleavage | 3.661 | 30.11 |
| TACI-14 Fc | No cleavage | 64.2 | 780.1 |

The sequences of further truncated TACI fragments are shown as follows:
> Amino acid sequence of TACI-16 (truncation at the C-terminus of TACI-9 by 3 amino acids "NKL" and at the N-terminus by 1 amino acid "S") (SEQ ID NO: 68): LSCRKEQGKFYDHLLRDCISCASICGQHPKQCAYFCE
> Amino acid sequence of TACI-17 (truncation at the C-terminus of TACI-9 by 3 amino acids "NKL" and at the N-terminus by 2 amino acids "SL") (SEQ ID NO: 69): SCRKEQGKFYDHLLRDCISCASICGQHPKQCAYFCE
> Amino acid sequence of TACI-18 (truncation at the C-terminus of TACI-9 by 3 amino acids "NKL" and at the N-terminus by 3 amino acids "SLS") (SEQ ID NO: 70): CRKEQGKFYDHLLRDCISCASICGQHPKQCAYFCE
> Amino acid sequence of TACI-19 (truncation at the C-terminus of TACI-9 by 3 amino acids "NKL" and at the N-terminus by 4 amino acids "SLSC") (SEQ ID NO: 71): RKEQGKFYDHLLRDCISCASICGQHPKQCAYFCE
> Amino acid sequence of TACI-20 (truncation at the C-terminus of TACI-9 by 3 amino acids "NKL" and at the N-terminus by 5 amino acids "SLSCR") (SEQ ID NO: 72): KEQGKFYDHLLRDCISCASICGQHPKQCAYFCE
> Amino acid sequence of TACI-21 (truncation at the C-terminus of TACI-9 by 3 amino acids "NKL" and at the N-terminus by 6 amino acids "SLSCRK") (SEQ ID NO: 73): EQGKFYDHLLRDCISCASICGQHPKQCAYFCE

The C-terminus of the above truncated TACI fragments was directly fused to the N-terminus of Fc (SEQ ID NO: 11) of human IgG to construct TACI-Fc fusion proteins, which were each transfected and expressed in 293E (human embryonic kidney cell modified by EBNA1 gene), and purified to obtain TACI-Fc fusion proteins comprising two identical polypeptides (see FIG. 1 for the structure).

The above-constructed TACI-Fc fusion proteins were analyzed by mass spectrometry. The experimental results are shown in Table 6-2. The experimental results showed that none of the TACI-Fc fusion proteins constructed by the truncated TACI-9 fragments had detectable TACI polypeptide cleavage.

In addition, the constructed TACI-Fc fusion proteins were assayed for the activity for binding to BAFF (see the ELISA binding assay (coating an antigen protein) of Test Example 1 of the present disclosure for the experimental method) and for the functional blocking for blocking the binding of BAFF to BAFF-R (see Test Example 2 of the present disclosure for the experimental method). The experimental results are shown in Table 6-2. The experimental results showed that the TACI-Fc fusion proteins constructed by the truncated TACI-9 fragments TACI-16, TACI-17, and TACI-18 of TACI-9 had good activity for binding to BAFF and functional activity for blocking the binding of BAFF to BAFF-R, while the TACI-Fc fusion protein constructed by the truncated fragments TACI-19, TACI-20, and TACI-21 had significantly weaker blocking activity.

**Table 6-2. Assay results for functional activity and cleavage by mass spectrometry of TACI-Fc fusion proteins**

| Fusion protein | TACI cleavage analysis | EC₅₀ (nM) for the binding of TACI-Fc to BAFF | IC₅₀ (nM) of TACI-Fc for blocking the binding of BAFF to BAFF-R |
|---|---|---|---|
| TACI-16Fc | No cleavage | 0.5987 | 2.224 |
| TACI-17Fc | No cleavage | 0.9958 | 2.824 |
| TACI-18Fc | No cleavage | 0.9924 | 3.256 |
| TACI-19Fc | No cleavage | 4.988 | 2219 |
| TACI-20Fc | No cleavage | 7.188 | 2361 |
| TACI-21Fc | No cleavage | 9.798 | 2524 |

### Example 3: TACI sequence engineering

TACI proteins are easy to aggregate in a neutral solution. To further improve the stability of TACI, the hydrophobic groups and ionic groups in TACI fragments were analyzed based on the crystal structure of TACI (PDB ID: 1XU1) by using MOE (molecular operating environment) software, and amino acid replacements were performed on amino acid residues at positions 2, 5, 6, 10, 18, 35, and/or 36 of TACI-9, so that the areas of the exposed hydrophobic groups and ionic groups of the TACI proteins were reduced, the aggregation of TACI was reduced, and the stability of TACI was improved while still basically remaining the functional activity of TACI. The amino acid sequences of the engineered TACI fragments are as follows:
> TACI-9-1 (TACI-9 with an L2T substitution) S**T**SCRKEQGKFYDHLLRDCISCASICGQHPKQCAYFCENKL
   SEQ ID NO: 18
> TACI-9-2 (TACI-9 with an R5S mutation) SLSC**S**KEQGKFYDBLLRDCISCASICGQUPKQCAYFCENKL
   SEQ ID NO: 19
> TACI-9-3 (TACI-9 with a K6E mutation) SLSCR**E**EQGKFYDHLLRDCISCASICGQHPKQCAYFCENKL
   SEQ ID NO: 20
> TACI-9-4 (TACI-9 with a K6Q mutation) SLSCR**Q**EQGKFYDHLLRDCISCASICGQHPKQCAYFCENKL
   SEQ ID NO: 21
> TACI-9-5 (TACI-9 with a K10E mutation) SLSCRKEQG**E**FYDHLLRDCISCASICGQHPKQCAYFCENKL
   SEQ ID NO: 22
> TACI-9-6 (TACI-9 with L2R and D18T mutations) S**R**SCRKEQGKFYDHLLR**T**CISCASICGQHPKQCAYFCENKL
   SEQ ID NO: 23
> TACI-9-7 (TACI-9 with L2R and D18A mutations) S**R**SCRKEQGKFYDHLLR**A**CISCASICGQHPKQCAYFCENKL
   SEQ ID NO: 24
> TACI-9-8 (TACI-9 with a Y35A mutation) SLSCRKEQGKFYDHLLRDCISCASICGQHPKQCA**A**FCENKL
   SEQ ID NO: 25
> TACI-9-9 (TACI-9 with L2R, D18T, and Y35R mutations) S**R**SCRKEQGKFYDHLLR**T**CISCASICGQHPKQCA**R**FCENKL
   SEQ ID NO: 26
> TACI-9-10 (TACI-9 with K6E and K10E mutations) SLSCR**E**EQG**E**FYDHLLRDCISCASICGQHPKQCAYFCENKL
   SEQ ID NO: 27
> TACI-9-11 (TACI-9 with R5S, K6E, and K10E mutations) SLSC**SE**EQG**E**FYDHLLRDCISCASICGQHPKQCAYFCENKL
   SEQ ID NO: 28
> TACI-9-12 (TACI-9 with L2T and Y35A mutations) S**T**SCRKEQGKFYDHLLRDCISCASICGQHPKQCA**A**FCENKL
   SEQ ID NO: 29
> TACI-9-13 (TACI-9 with L2T and F36Y mutations) STSCRKEQGKFYDHLLRDCISCASICGQHPKQCAYFCENKL
   SEQ ID NO: 30
> TACI-9-14 (TACI-9 with L2T, Y35A, and F36Y mutations) S**T**SCRKEQGKFYDHLLRDCISCASICGQHPKQCA**AY**CENKL
   SEQ ID NO: 31
> TACI-9-15 (TACI-9 with L2T, K6E, K10E, and Y35A mutations) S**T**SCR**E**EQG**E**FYDHLLRDCISCASICGQHPKQCA**A**FCENKL
   SEQ ID NO: 32
Note: In the above sequences, the underlined parts indicate amino acid residues after mutation.

The C-terminus of the above TACI fragments obtained by the engineering of the TACI-9 was fused to the N-terminus of Fc (SEQ ID NO: 11) of human IgG to construct TACI-Fc fusion proteins (see FIG. 1 for the structure of the constructed fusion proteins), which were transfected and expressed in 293E (human embryonic kidney cells modified by EBNA1 gene), and purified by Protein A affinity chromatography to obtain TACI-Fc fusion proteins comprising two identical polypeptides.

The constructed TACI-Fc fusion proteins were assayed for the activity for binding to BAFF (see ELISA binding assay (coating an antigen protein) of Test Example 1 of the present disclosure for the experimental method). The experimental results are shown in Table 7:

**Table 7. Activity of TACI-Fc fusion proteins for binding to BAFF**

| TACI-Fc fusion protein | Mutation positions in TACI sequence | EC₅₀ (nM) for the binding of TACI-Fc fusion protein to BAFF |
|---|---|---|
| TACI-9Fc | None | 0.4181 |
| TACI-9-1Fc | L2T | 0.3820 |
| TACI-9-2Fc | R5S | 0.2785 |
| TACI-9-3Fc | K6E | 0.2654 |
| TACI-9-4Fc | K6Q | 0.4695 |
| TACI-9-5Fc | K10E | 0.1456 |
| TACI-9-6Fc | L2R,D18T | 0.3672 |
| TACI-9-7Fc | L2R,D18A | 0.7273 |
| TACI-9-8Fc | Y35A | 0.1999 |
| TACI-9-9Fc | L2R,D18T,Y35R | 0.9368 |
| TACI-9-10Fc | K6E,K10E | 0.1199 |
| TACI-9-11Fc | R5S,K6E,K10E | 0.1950 |
| TACI-9-12Fc | L2T, Y35A | 0.1223 |
| TACI-9-13Fc | L2T, F36Y | 0.4327 |
| TACI-9-14Fc | L2T, Y35A,F36Y | 0.2233 |
| TACI-9-15Fc | L2T,K6E,K10E,Y35A | 0.0869 |

Note: The mutation positions in the table are amino acid residue positions (numbered in natural order) relative to TACI-9. For example, "L2T" indicates that the amino acid residue at position 2 (numbered in natural order) of the sequence SEQ ID NO: 10 is mutated from the original "L" to "T".

In addition, part of the TACI-Fc fusion proteins obtained by purification were each buffer-exchanged into a PBS solution at pH 7.4 using an ultrafiltration tube, and the appearance of the solution was observed to determine whether there was precipitation in the TACI-Fc fusion protein solution. The experimental results are shown in Table 8. The experimental results showed that there was no precipitation in the solution of the TACI-Fc fusion proteins constructed by the engineered TACI fragments, while there was precipitation in a solution of RCT-18 in PBS.

**Table 8. Precipitation of TACI-Fc fusion proteins in PBS solution**

| TACI-Fc fusion protein | Whether or not there is precipitation |
|---|---|
| TACI-9-2Fc | No |
| TACI-9-3Fc | No |
| TACI-9-4Fc | No |
| TACI-9-5Fc | No |
| TACI-9-8Fc | No |
| TACI-9-10Fc | No |
| TACI-9-11Fc | No |
| TACI-9-12Fc | No |
| TACI-9-13Fc | No |
| TACI-9-14Fc | No |
| TACI-9-15Fc | No |
| RCT-18 | Yes |

In addition, PI values of part of the TACI-Fc fusion proteins were analyzed by the "18cProt/TrEMBL" system, with the analysis results shown in Table 9:

**Table 9. pI values of TACI-Fc fusion proteins**

| TACI-Fc fusion protein | pI value |
|---|---|
| TACI-9 Fc | 8.3 |
| TACI-9-3 Fc | 7.3 |
| TACI-9-5 Fc | 7.3 |
| TACI-9-10 Fc | 5.7 |
| TACI-9-11 Fc | 5.0 |
| TACI-9-15 Fc | 5.7 |

In addition, the TACI-Fc fusion proteins at a concentration of 40 mg/mL were each buffer-exchanged into a PBS solution at pH 7.4, the solution was placed in a constant-temperature incubator at 40 °C for an accelerated stability test, in which samples were taken every week for SEC purity analysis, with the experimental result shown in Table 10. The experimental result showed that the TACI-9-15Fc fusion protein had the best stability performance, which, after being stored at a constant temperature of 40 °C for at least four weeks, still had a purity of more than 94%.

**Table 10. Change in SEC purity of TACI-Fc fusion proteins in PBS solution**

| Sample name | | TACI-9Fc | TACI-9-10Fc | TACI-9-15Fc |
|---|---|---|---|---|
| SEC purity | At the beginning | 97.14% | 85.38% | 96.52% |
| | 1 week | 59.18% | 83.27% | 95.66% |
| | 2 weeks | 57.13% | 83.11% | 95.19% |
| | 3 weeks | 55.99% | 82.73% | 95.00% |
| | 4 weeks | 48.37% | 80.23% | 94.54% |

### Example 4: Construction of TACI-Fc fusion proteins

TACI-9-15 or truncated forms thereof (TACI-9-15a, TACI-9-15b, and TACI-9-15c) were fused to Fc of human IgG to construct bivalent or tetravalent TACI-Fc fusion proteins. The schematic structural diagrams of the constructed TACI-Fc fusion proteins are shown in FIGs. 1, 2, and 3.
> Sequence of TACI-9-15a STSCREEQGEFYDHLLRDCISCASICGQHPKQCAAFCENK
   SEQ ID NO: 33
> Sequence of TACI-9-15b STSCREEQGEFYDHLLRDCISCASICGQHPKQCAAFCEN
   SEQ ID NO: 34
> Sequence of TACI-9-15c STSCREEQGEFYDHLLRDCISCASICGQHPKQCAAFCE
   SEQ ID NO: 35

The full-length sequences of the constructed TACI-Fc fusion proteins are as follows:
> Sequence of TACI-9-15Fc1 fusion protein 
STSCREEQGEFYDHLLRDCISCASICGQHPKQCAAFCENKLEPKSSDKTHTCPPC
PAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSL
SPGK
SEQ ID NO: 36
> Sequence of TACI-9-15Fc2 fusion protein 
STSCREEQGEFYDHLLRDCISCASICGQHPKQCAAFCENEPKSSDKTHTCPPCPA
PELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPG
K
SEQ ID NO: 37
> Sequence of TACI-9-15Fc3 fusion protein 
STSCREEQGEFYDHLLRDCISCASICGQHPKQCAAFCEEPKSSDKTHTCPPCPAP
ELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPG
K
SEQ ID NO: 38
> Sequence of TACI-9-15Fc4 fusion protein DKTHTCPPCPAPELLGGPSVFLFPPKPKDTLMISRTPEVTCVVVDVSHEDPEVKF 
NWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNH
YTQKSLSLSPGGGGSSTSCREEQGEFYDHLLRDCISCASICGQHPKQCAAFCEN
K
SEQ ID NO: 39
> Sequence of TACI-9-15Fc5 fusion protein 
STSCREEQGEFYDHLLRDCISCASICGQHPKQCAAFCEDKTHTCPPCPAPEAEGA
PSVFLFPPKPKDTLMISRTPEVTCVVVDVSHEDPEVKFNWYVDGVEVHNAKTK
PREEQYNSTYRVVSVLTVLHQDWLNGKEYKCKVSNKALPSSIEKTISKAKGQPR
EPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
SEQ ID NO: 40
> Sequence of TACI-9-15Fc6 fusion protein 
STSCREEQGEFYDHLLRDCISCASICGQHPKQCAAFCEEPKSSDKTHTCPPCPAP
EAEGAPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPSSIEKTISK
AKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPG
K
SEQ ID NO: 41
> Sequence of TACI-9-15Fc7 fusion protein 
STSCREEQGEFYDHLLRDCISCASICGQHPKQCAAFCEEPKSSDKTHTCPPCPAP
EAEGAPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPSSIEKTISK
AKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPG
GGGSSTSCREEQGEFYDHLLRDCISCASICGQHPKQCAAFCEQK
SEQ ID NO: 42
> Sequence of TACI-9-15Fc8 fusion protein 
STSCREEQGEFYDHLLRDCISCASICGQHPKQCAAFCEEPKSSDKTHTCPPCPAP
EAEGAPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPSSIEKTISK
AKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPG
GGGSSTSCREEQGEFYDHLLRDCISCASICGQHPKQCAAFCESK
SEQ ID NO: 43
> Sequence of TACI-9-15Fc9 fusion protein

STSCREEQGEFYDHLLRDCISCASICGQHPKQCAAFCEEPKSSDKTHTCPPCPAP
EAEGAPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPSSIEKTISK

AKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPG
GGGSSTSCREEQGEFYDHLLRDCISCASICGQHPKQCAAFCESS
SEQ ID NO: 44

Note: In the full-length sequence of the TACI-Fc fusion protein, the single underlined part indicates a TACI sequence, the wavy line part indicates an Fc sequence of human IgG, the bold font part indicates a linker sequence and the italic font part indicates a protecting amino acid.

### Exemplary Fc sequences:

> Fc sequence 1 of human IgG 
DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNH
YTQKSLSLSPGK
SEQ ID NO: 61
> Fc sequence 2 of human IgG 
DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNH
YTQKSLSLSPG
SEQ ID NO: 62
> Fc sequence 3 of human IgG

DKTHTCPPCPAPEAEGAPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPSSIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNH
YTQKSLSLSPG
SEQ ID NO: 63

### Exemplary linker sequences:

> Linker sequence 1 EPKSS SEQ ID NO: 64
> Linker sequence 2 GGGS SEQ ID NO: 65
> Linker sequence 3 GGGGS SEQ ID NO: 66
> Linker sequence 4 GGGGSGGGGSGGGGS SEQ ID NO: 67

### Example 5: Construction of TACI antibody fusion proteins

The light and/or heavy chains of an anti-IL-12/IL-23 p40 subunit antibody ustekinumab were fused to a TACI polypeptide to construct a TACI antibody fusion protein TACI-Mab.

The sequences of the ustekinumab antibody are shown below:
> Heavy chain variable region of ustekinumab 
EVQLVQSGAEVKKPGESLKISCKGSGYSFTTYWLGWVRQMPGKGLDWIGIMSP
VDSDIRYSPSFQGQVTMSVDKSITTAYLQWNSLKASDTAMYYCARRRPGQGYF
DFWGQGTLVTVSS
SEQ ID NO: 45
> Light chain variable region of ustekinumab 
DIQMTQSPSSLSASVGDRVTITCRASQGISSWLAWYQQKPEKAPKSLIYAASSLQ
SGVPSRFSGSGSGTDFTLTISSLQPEDFATYYCQQYNIYPYTFGQGTKLEIK
SEQ ID NO: 46
> Heavy chain of ustekinumab 
EVQLVQSGAEVKKPGESLKISCKGSGYSFTTYWLGWVRQMPGKGLDWIGIMSP
VDSDIRYSPSFQGQVTMSVDKSITTAYLQWNSLKASDTAMYYCARRRPGQGYF
DFWGQGTLVTVSSSTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
RVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 47
> Light chain of ustekinumab

DIQMTQSPSSLSASVGDRVTITCRASQGISSWLAWYQQKPEKAPKSLIYAASSLQ
SGVPSRFSGSGSGTDFTLTISSLQPEDFATYYCQQYNIYPYTFGQGTKLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 48

Note: in the above sequences of the heavy and light chains of ustekinumab, the double-underlined part indicates a variable region sequence, and the non-underlined part indicates a constant region sequence.

The CDR sequences of ustekinumab are shown in Table 11:

**Table 11. Antibody CDR sequences of ustekinumab**

| | | | |
|---|---|---|---|
| LCDR1 | RASQGISSWLA (SEQ ID NO: 49) | HCDR1 | TYWLG (SEQ ID NO: 52) |
| LCDR2 | AASSLQS (SEQ ID NO: 50) | HCDR2 | IMSPVDSDIRYSPSFQG(SEQ ID NO: 53) |
| LCDR3 | QQYNIYPYT (SEQ ID NO: 51) | HCDR3 | RRPGQGYFDF (SEQ ID NO: 54) |

| | | | |
|---|---|---|---|
| Note: the above CDRs are determined according to the Kabat numbering scheme. | | | |

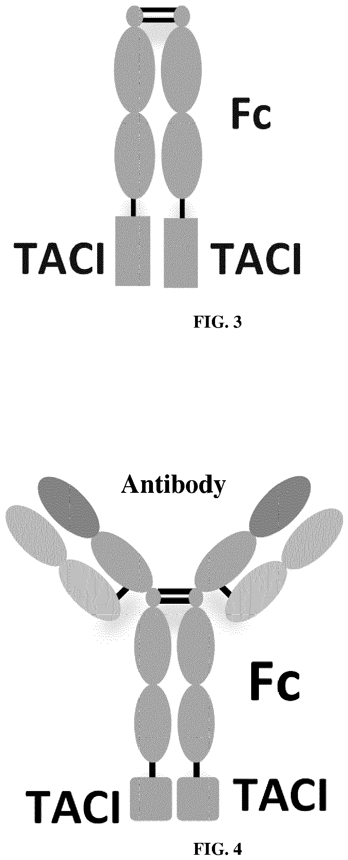

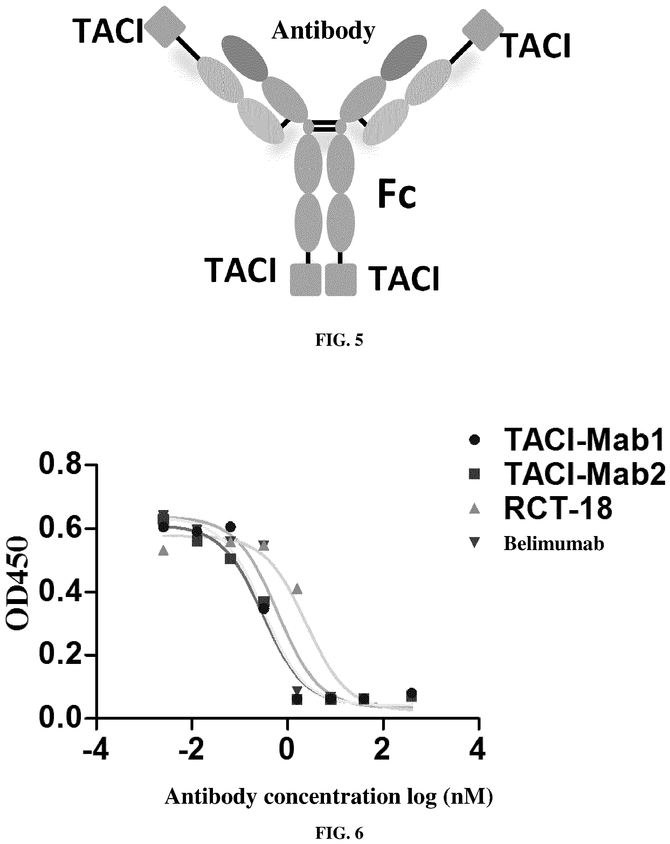

TACI was fused to the heavy and/or light chains of ustekinumab to construct antibody fusion proteins TACI-Mab1 (see FIG. 4 for a schematic structural diagram) and TACI-Mab2 (see FIG. 5 for a schematic structural diagram). The sequences are as follows:
> Heavy chain of TACI-Mab 1 
EVQLVQSGAEVKKPGESLKISCKGSGYSFTTYWLGWVRQMPGKGLDWIGIMSP
VDSDIRYSPSFQGQVTMSVDKSITTAYLQWNSLKASDTAMYYCARRRPGQGYF
DFWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGGGGSSTSCREEQGEFYDHLLRDCISCASICGQHPKQ
CAAFCENK
SEQ ID NO: 55
> Light chain of TACI-Mab 1 
DIQMTQSPSSLSASVGDRVTITCRASQGISSWLAWYQQKPEKAPKSLIYAASSLQ
SGVPSRFSGSGSGTDFTLTISSLQPEDFATYYCQQYNIYPYTFGQGTKLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 56
> Heavy chain of TACI-Mab2 
EVQLVQSGAEVKKPGESLKISCKGSGYSFTTYWLGWVRQMPGKGLDWIGIMSP
VDSDIRYSPSFQGQVTMSVDKSITTAYLQWNSLKASDTAMYYCARRRPGQGYF
DFWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGGGGSSTSCREEQGEFYDHLLRDCISCASICGQHPKQ
CAAFCENK
SEQ ID NO: 57
> Light chain of TACI-Mab2

STSCREEQGEFYDHLLRDCISCASICGQHPKQCAAFCENKLGGGGSGGGGSGG
GGSDIQMTQSPSSLSASVGDRVTITCRASQGISSWLAWYQQKPEKAPKSLIYAAS
SLQSGVPSRFSGSGSGTDFTLTISSLQPEDFATYYCQQYNIYPYTFGQGTKLEIKR
TVAAPSVFIFPPSDEQLKSGTASVVCLLNNFYPREAKVQWKVDNALQSGNSQES

VTEQDSKDSTYSLSSTLTLSKADYEKHKVYACEVTHQGLSSPVTKSFNRGEC
SEQ ID NO: 58

Note: In the light chain and heavy chain sequences of the antibody fusion protein, the single underlined part indicates a TACI sequence, the double-underlined part indicates a variable region sequence, the wavy line part indicates a constant region sequence, and the bold front part indicates a linker sequence.

The marketed anti-BAFF antibody belimumab was used as a control antibody. The sequences of the belimumab antibody are described in WO 2015173782A1, specifically shown as follows:
> Heavy chain of belimumab 
QVQLQQSGAEVKKPGSSVRVSCKASGGTFNNNAINWVRQAPGQGLEWMGGIIP
MFGTAKYSQNFQGRVAITADESTGTASMELSSLRSEDTAVYYCARSRDLLLFPH
HALSPWGRGTMVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSC
SVMHEALHNHYTQKSLSLSPGK
SEQ ID NO: 59
> Light chain of belimumab

SSELTQDPAVSVALGQTVRVTCQGDSLRSYYASWYQQKPGQAPVLVIYGKNNRP
SGIPDRFSGSSSGNTASLTITGAQAEDEADYYCSSRDSSGNHWVFGGGTELTVLG
QPKAAPSVTLFPPSSEELQANKATLVCLISDFYPGAVTVAWKADSSPVKAGVETT
TPSKQSNNKYAASSYLSLTPEQWKSHRSYSCQVTHEGSTVEKTVAPTECS
SEQ ID NO: 60

### Test Example 1: ELISA binding assay

### I. ELISA binding assay (coating an antigen protein)

The binding activity of the antibodies or fusion proteins for BAFF, APRIL, and IL-12/23 p40 was assayed by the ELISA method (coating an antigen protein). The specific procedures were as follows:
A BAFF (Sino biological, 10056-HNCH), APRIL (R&D Systems, 5860-AP-010/CF), or IL-12/23 p40 (Sino biological, 10052-H08H) protein was diluted to 1 µg/mL with a PBS (BasalMedia, B320) buffer at pH 7.4, and added to a 96-well microplate (Corning, 3590) at 100 µL/well. The plate was incubated at 4 °C overnight. After the liquid was discarded, 300 µL of 5% skim milk (BD, 232100) diluted with PBS was added to each well for blocking, and the plate was incubated at 37 °C for 2 h. After the blocking was completed, the blocking solution was discarded, and the plate was washed 3 times with a PBST buffer (PBS at pH 7.4 containing 0.1% tween-20). 100 µL of test antibody or fusion protein solution diluted in a gradient was added to each well, and the plate was incubated at 37 °C for 1 h. After the incubation was completed, the plate was washed 3 times with PBST. 100 µL of mouse anti-human IgG (H+L) (Jackson ImmunoResearch, 209-035-088, diluted at a ratio of 1:8000) was added to each well, and the plate was incubated at 37 °C for 1 h. The plate was washed 3 times with PBST. 100 µL of TMB chromogenic substrate (KPL, 5120-0077) was added to each well, and the plate was incubated at room temperature for 10-15 min. The reaction was stopped by adding 50 µL of 1 M H₂SO₄ to each well. The plate was read for absorbance values at 450 nm on a microplate reader, the binding curves of the antibodies to an antigen were fitted with software, and the EC₅₀ values were calculated. The experimental results are shown in Tables 12-14 below. The experimental results showed that TACI-Mab1 and TACI-Mab2 had good binding activity to all of BAFF, APRIL, and IL-12/23 p40, and had EC₅₀ values for binding to BAFF less than that of RCT-18 and EC₅₀ values for binding to IL-12/23 p40 less than that of ustekinumab.

**Table 12. Experimental results for the binding of antibody fusion proteins to BAFF**

| Sample | EC₅₀ (nM) for binding to BAFF |
|---|---|
| TACI-Mab1 | 3.748 |
| TACI-Mab2 | 4.788 |
| RCT-18 | 6.713 |
| Ustekinumab | No binding |

**Table 13. Experimental results for the binding of antibody fusion proteins to APRII,**

| Sample | EC₅₀ (nM) for binding to APRIL |
|---|---|
| TACI-Mab1 | 1.844 |
| TACI-Mab2 | 1.291 |
| Belimumab | No binding |
| Ustekinumab | No binding |

**Table 14. Experimental results for the binding of antibody fusion proteins to IL-12/23 p40**

| Sample | EC₅₀ (nM) for binding to IL-12/23 p40 |
|---|---|
| TACI-Mab1 | 0.6187 |
| TACI-Mab2 | 0.5421 |
| RCT-18 | No binding |
| Belimumab | No binding |
| Ustekinumab | 0.7856 |

### II. ELISA binding assay (coating a test sample)

The binding activity of the TACI fusion proteins for BAFF and APRII, was assayed by the ELISA method (coating a test fusion protein sample). The specific procedures were as follows:
The test sample was diluted to 2 µg/mL with a PBS (BasalMedia, B320) buffer at pH 7.4 and added to a 96-well microplate (Corning, 3590) at 100 µL/well. The plate was incubated at 4 °C overnight. After the liquid was discarded, 300 µL of 5% skim milk (BD, 232100) diluted with PBS was added to each well for blocking, and the plate was incubated at 37 °C for 2 h. After the blocking was completed, the blocking solution was discarded, and the plate was washed 3 times with a PBST buffer (PBS at pH 7.4 containing 0.1% tween-20). 100 µL of BAFF (ACROBiosystems, BAF-H5248) or APRIL (R&D Systems, 5860-AP-010/CF) diluted in a gradient was added to each well, and the plate was incubated at 37 °C for 1 h. After the incubation, the plate was washed 3 times with PBST. When detecting the binding to BAFF, 100 µL of Anti-His-HRP (Sino biological, 105327-MM02T-H, diluted at a ratio of 1:2000) was added to each well; when detecting the binding to APRIL, 100 µL of anti-HA-HRP (Abcam, ab1190, diluted at a ratio of 1:4000) was added to each well. The plate was incubated at 37 °C for 1 h. The plate was washed 3 times with PBST. 100 µL of TMB chromogenic substrate (KPL, 5120-0077) was added to each well, and the plate was incubated at room temperature for 10-15 min. The reaction was stopped by adding 50 µL of 1 M H₂SO₄ to each well. The plate was read for absorbance values at 450 nm on a microplate reader, the binding curves of the antibodies to an antigen were fitted with software, and the EC₅₀ values were calculated. The experimental results are shown in Table 15 below. The experimental results showed that the activity of the TACI-Fc fusion proteins constructed by the present disclosure for binding to BAFF was significantly stronger than that of the control RCT-18.

**Table 15. Experimental results for the binding of TACI-Fc fusion proteins to BAFF**

| TACI fusion protein | EC₅₀ (nM) for binding to BAFF |
|---|---|
| TACI-9-15Fc1 | 7.023 |
| TACI-9-15Fc2 | 8.801 |
| TACI-9-15Fc3 | 9.072 |
| TACI-9-15Fc4 | 20.09 |
| TACI-9-15Fc5 | 8.889 |
| TACI-9-15Fc6 | 8.477 |
| TACI-9-15Fc7 | 8.952 |
| TACI-9-15Fc8 | 10.48 |
| TACI-9-15Fc9 | 8.940 |
| RCT-18 | 96.2 |

### Test Example 2: Assay on the blocking of ligand and receptor

The blocking activity of the antibodies and the fusion proteins for receptors and ligands such as BAFF/BAFF-R, BAFF/BCMA, BAFF/TACI, APRII,/BCMA, APRIL/TACI, and p40/IL-12Rβ1 was assayed by the ELISA method. The specific procedures were as follows:
A receptor protein was diluted to 2 µg/mL with PBS at pH 7.4 (BasalMedia, B320) and added to a 96-well microplate (Corning, 3590) at 100 µL/well. The plate was incubated at 4 °C overnight. After the liquid was discarded, 200 µL of 1% Casein blocking solution (Thermo, 37528) was added to each well for blocking, and the plate was incubated at 37 °C for 2 h. After the blocking was completed, the blocking solution was discarded, and the plate was washed 3 times with a PBST buffer (PBS at pH 7.4 containing 0.1% tween-20) for later use. A biotin-labeled ligand protein at a fixed concentration was mixed with the antibody or fusion protein diluted in gradient, pre-incubated at 37 °C for 30 min, and added to a blocked microplate. The plate was incubated at 37 °C for 1.5 h. After the incubation was completed, the plate was washed 3 times with PBST. 100 µL of streptavidin-HRP (Invitrogen, 434323, diluted at a ratio of 1:4000) was added to each well, and the plate was incubated at 37 °C for 1 h. The supernatant was removed, and the plate was washed 3 times with PBST. 100 µL of TMB chromogenic substrate (KPL, 5120-0077) was added to each well, and the plate was incubated at room temperature for 10-15 min. The reaction was stopped by adding 50 µL of 1 M H₂SO₄ to each well. The plate was read for absorbance values at 450 nm on a microplate reader, the curves for the inhibition of the binding of the ligands to the receptors were fitted with software, and the IC₅₀ values were calculated.

The source information of the ligand and receptor proteins used in this test example is as follows: BAFF (Sino biological, 10056-HNCH), APRIL (R&D Systems, 5860-AP-010/CF), p40 (Sino biological, 10052-H08H), BAFF-R (Sino biological, 16079-H02H), BCMA (Sino biological, 10620-H02H), TACI (ACROBiosystems, TAI-H5256), IL-12Rβ1 (ACROBiosystems, ILB-H5255).

The experimental results for the blocking of the binding function of BAFF to BAFF-R by the fusion proteins of the present disclosure are shown in Table 16 below. The experimental results showed that the activity of the TACI fusion proteins constructed by the present disclosure for blocking the binding of BAFF to BAFF-R was stronger than that of the control RCT-18.

**Table 16. Experimental results for the blocking of the binding of BAFF to BAFF-R by the TACI fusion proteins**

| IC₅₀ (nM) of the TACI fusion proteins | for blocking the binding of BAFF to BAFF-R |
|---|---|
| TACI-9-15Fc1 | 2.621 |
| TACI-9-15Fc2 | 1.355 |
| TACI-9-15Fc3 | 1.066 |
| TACI-9-15Fc4 | 1.232 |
| TACI-9-15Fc5 | 1.439 |
| TACI-9-15Fc6 | 1.872 |
| TACI-9-15Fc7 | 1.379 |
| TACI-9-15Fc8 | 1.559 |
| TACI-9-15Fc9 | 1.185 |
| RCT-18 | 19.24 |

For the antibody fusion proteins of the present disclosure, the experimental results for blocking the binding of BAFF to BAFF-R are shown in FIG. 6, the experimental results for blocking the binding of BAFF to BCMA are shown in Table 17, the experimental results for blocking the binding of BAFF to TACI are shown in Table 18, the experimental results for blocking the binding of APRIL to BCMA are shown in Table 19, the experimental results for blocking the binding of APRIL to TACI are shown in Table 20, and the experimental results for blocking the binding of p40 to IL-12Rβ1 are shown in Table 21. The experimental result showed that the TACI antibody fusion proteins constructed by the present disclosure were able to effectively block the binding of related receptors to ligands thereof, and had stronger activity for blocking the binding of BAFF to BAFF-R, BAFF to BCMA, BAFF to TACI, APRIL to BCMA, and APRIL to TACI than the control; the TACI antibody fusion proteins constructed by the present disclosure were able to effectively block the binding of p40 to IL-12Rβ1, while RCT-18 and belimumab did not have such blocking activity.

**Table 17. Experimental results for the blocking of the binding of BAFF to BCMA by the TACI antibody fusion proteins**

| Sample | IC₅₀ (nM) for blocking the binding of BAFF to BCMA |
|---|---|
| TACI-Mab 1 | 0.1429 |
| TACI-Mab2 | 0.1947 |
| RCT-18 | 0.9725 |
| Belimumab | 0.4584 |
| Ustekinumab | No blocking activity |

**Table 18. Experimental results for the blocking of the binding of BAFF to TACI by the TACI antibody fusion proteins**

| Sample | IC₅₀ (nM) for blocking the binding of BAFF to TACI |
|---|---|
| TACI-Mab 1 | 0.2525 |
| TACI-Mab2 | 0.1841 |
| RCT-18 | 0.7788 |
| Belimumab | 0.5666 |
| Ustekinumab | No blocking activity |

**Table 19. Experimental results for the blocking of the binding of APRIL to BCMA by the TACI antibody fusion proteins**

| Sample | IC₅₀ (nM) for blocking the binding of APRIL to BCMA |
|---|---|
| TACI-Mab2 | 2.431 |
| RCT-18 | 25.12 |
| Belimumab | No blocking activity |
| Ustekinumab | No blocking activity |

**Table 20. Experimental results for the blocking of the binding of APRIL to TACI by the TACI antibody fusion proteins**

| Sample | IC₅₀ (nM) for blocking the binding of APRIL to TACI |
|---|---|
| TACI-Mab2 | 0.8334 |
| RCT-18 | 3.297 |
| Belimumab | No blocking activity |
| Ustekinumab | No blocking activity |

**Table 21. Experimental results for the blocking of the binding of p40 to IL-12Rβ1 by the TACI antibody fusion proteins**

| Sample | IC₅₀ (nM) for blocking the binding of p40 to IL-12Rβ1 |
|---|---|
| TACI-Mab 1 | 2.063 |
| TACI-Mab2 | 2.085 |
| RCT-18 | No blocking activity |
| Belimumab | No blocking activity |
| Ustekinumab | 2.077 |

### Test Example 3: Cell proliferation assay

A B cell proliferation assay was performed to determine whether the antibodies and the fusion proteins were able to inhibit the B cell proliferation induced by BAFF and APRIL. The specific procedures were as follows:
The spleens of mice were ground and centrifuged at 4 °C for 5 min, and lower-layer cells were collected, washed once with a washing solution (PBS + 2% FBS + 2 mM EDTA), and centrifuged. The supernatant was removed, an RBC lysis buffer (Invitrogen, 00-4333-57) was added, and the mixture was left to stand at room temperature for 5 min until the red blood cells were completely lysed. The lysate was centrifuged, and the cells were resuspended and counted. The cell suspension was sorted using B Cell Isolation Kit (Miltenyi Biotec, 130-090-862), and the isolated B cells were resuspended in RPMI 1640 Medium (Gibco, 11875119) + 10% FBS (Gibco, 10099-141) + 50 µM 2-mercaptoethanol (Sigma-Aldrich, M6250) and counted. The cells were seeded into a 96-well cell plate (Costar, 3903) for later use. The BAFF (R&D Systems, 7537-BF) or APRIL (R&D Systems, 5860-AP) protein was diluted to a fixed concentration, and the antibody or fusion protein diluted in a gradient was added. The mixture was mixed homogeneously, incubated at 37 °C for 30 min, added to a 96-well cell plate, and then cultured in a cell incubator at 37 °C for 48 h. The cell culture plate was taken out, and 50 µL of Celltiter Glo detection solution (Promega, G7573) was added to each well. The plate was incubated at room temperature for 10 min. The bioluminescent signals were detected by a microplate reader, the detection results were fitted to obtain an inhibition curve using software, and the IC₅₀ values were calculated.

The experimental results for the activity of the TACI antibody fusion proteins of the present disclosure for inhibiting the BAFF-induced B cell proliferation are shown in Table 22 below. The experimental results showed that the TACI-Mab 1 and TACI-Mab2 of the present disclosure had the strongest activity for inhibiting the BAFF-induced B cell proliferation, belimumab had the second strongest activity, the RCT-18 had weaker activity, and the ustekinumab had no activity for inhibiting the BAFF-induced B cell proliferation.

**Table 22. Experimental results for the inhibition of the BAFF-induced B cell proliferation by the TACI antibody fusion proteins**

| Sample | IC₅₀ (nM) for the inhibition of the BAFF-induced B cell proliferation |
|---|---|
| TACI-Mab1 | 0.06715 |
| TACI-Mab2 | 0.05711 |
| RCT-18 | 0.386 |
| Belimumab | 0.1464 |
| Ustekinumab | No inhibitory activity |

The experimental results for the activity of the TACI antibody fusion proteins of the present disclosure for inhibiting the APRIL-induced B cell proliferation are shown in Table 23 below. The experimental results showed that the activity of the TACI-Mab2 of the present disclosure for inhibiting the APRIL-induced B cell proliferation was increased by 12.2 times as compared to that of RCT-18, while belimumab and ustekinumab had no activity for inhibiting the APRIL-induced B cell proliferation.

**Table 23. Experimental results for the inhibition of the APRII,-induced B cell proliferation by the TACI antibody fusion proteins**

| Sample | IC₅₀ (nM) for the inhibition of the APRIL-induced B cell proliferation |
|---|---|
| TACI-Mab2 | 0.08921 |
| RCT-18 | 1.087 |
| Belimumab | No inhibitory activity |
| Ustekinumab | No inhibitory activity |

The experimental results for the activity of the TACI-Fc fusion proteins of the present disclosure for inhibiting the B cell proliferation induced by BAFF or APRIL are shown in Tables 24 and 25. The experimental results showed that the TACI fusion proteins constructed by the present disclosure were able to effectively inhibit the BAFF-induced B cell proliferation activity, and were also able to effectively inhibit the APRIL-induced B cell proliferation activity.

**Table 24. Experimental results for the inhibition of the BAFF-induced B cell proliferation by the TACI-Fc fusion proteins**

| TACI fusion protein | IC₅₀ (nM) for the inhibition of the BAFF-induced B cell proliferation |
|---|---|
| RCT-18 | 0.3815 |
| TACI-9-15Fc2 | 0.054 |
| TACI-9-15Fc6 | 0.04808 |
| TACI-9-15Fc7 | 0.02436 |
| TACI-9-15Fc8 | 0.02911 |
| TACI-9-15Fc9 | 0.02623 |

**Table 25. Experimental results for the inhibition of the APRIL-induced B cell proliferation by the TACI-Fc fusion proteins**

| TACI fusion protein | IC₅₀ (nM) for the inhibition of the APRIL-induced B cell proliferation |
|---|---|
| TACI-9-15Fc2 | 0.2305 |
| TACI-9-15Fc6 | 0.1400 |
| TACI-9-15Fc7 | 0.004805 |
| TACI-9-15Fc8 | 0.004767 |
| TACI-9-15Fc9 | 0.009472 |

### Test Example 4: IFNγ and IL-17 secretion assay

**IFNγ** and IL-17 secretion assays were performed to determine whether the antibodies and the fusion proteins were able to inhibit the T cell differentiation induced by IL-12 and IL-23. The specific procedures were as follows:
100 µL of 2 µg/mL anti-mouse CD3 antibody (BioLegend, 100238) and 2 µg/mL anti-mouse CD28 antibody (BioLegend, 102116) were added to each well of a 96-well plate (Corning, 3599). The plate was incubated at 37 °C for 1 h and washed twice with PBS for later use. The spleens of mice were ground and centrifuged at 4 °C for 5 min, and lower-layer cells were collected, washed once with a washing solution (PBS + 2% FBS + 2 mM EDTA), and centrifuged. The supernatant was removed, an RBC lysis buffer (Invitrogen, 00-4333-57) was added, and the mixture was left to stand at room temperature for 5 min until the red blood cells were completely lysed. The lysate was centrifuged, and the cells were resuspended and counted. The cell suspension was sorted with the Mouse CD4 Cells Kit (Invitrogen, 11415D), and the isolated CD4⁺ T cells were resuspended in a medium of RPMI 1640 Medium (Gibco, 11875119) + 10% FBS (Gibco, 10099-141) and counted for later use.

When detecting the T cell differentiation induced by IL-12, 20 µg/mL anti-mouse IL-4 (BioLegend, 504122) was added to T cells, and the cell suspension was applied to a coated 96-well plate. The chimeric IL-12 (human p40-mouse p35) protein at a fixed concentration was mixed with the antibody or fusion protein diluted in a gradient, pre-incubated at 37 °C for 1 h, added to the 96-well plate, and cultured in a cell incubator at 37 °C for 48 h. The 96-well plate was taken out and centrifuged at 1000 rpm for 3 min. The supernatant was collected and assayed for IFNγ content using the Mouse IFN-gamma DuoSet ELISA Kit (R&D Systems, DY485).

When detecting the T cell differentiation induced by IL-23, the cell suspension was applied to a coated 96-well plate. IL-23 (R&D Systems, 1290-IL-010) at a fixed concentration was mixed with the antibody or fusion protein diluted in a gradient, pre-incubated for 1 h, added to the 96-well plate, and cultured in a cell incubator at 37 °C for 48 h. The 96-well plate was taken out and centrifuged at 1000 rpm for 3 min. The supernatant was collected and assayed for IL-17 content using the Mouse IL-17 DuoSet ELISA Kit (R&D Systems, DY421).

The experimental results for the inhibition of the IL-23-induced IL-17 secretion by the TACI antibody fusion proteins of the present disclosure are shown in Table 26 below. The experimental results showed that RCT-18 and belimumab had no function of inhibiting the IL-23-induced IL-17 secretion, while the TACI-Mab1 and TACI-Mab2 of the present disclosure were able to effectively inhibit the IL-23-induced IL-17 secretion, with the IC₅₀ values less than that of ustekinumab.

**Table 26. Experimental results for the inhibition of IL-23-induced IL-17 secretion by the TACI antibody fusion proteins**

| Sample | IC₅₀ (nM) for the inhibition of IL-23-induced IL-17 secretion |
|---|---|
| TACI-Mab1 | 1.693 |
| TACI-Mab2 | 2.339 |
| RCT-18 | No inhibitory activity |
| Belimumab | No inhibitory activity |
| Ustekinumab | 2.487 |

The experimental results for the inhibition of the IL-12-induced **IFNγ** secretion by the TACI antibody fusion proteins of the present disclosure are shown in Table 27 below. The experimental results showed that the TACI-Mab 1 and TACI-Mab2 of the present disclosure were able to effectively inhibit the IL-12-induced **IFNγ** secretion, while RCT-18 and belimumab both had no activity for inhibiting the IL-12-induced **IFNγ** secretion.

**Table 27. Experimental results for the inhibition of IL-12-induced IFNγ secretion by the TACI antibody fusion proteins**

| Sample | IC₅₀ (nM) for the inhibition of IL-12-induced **IFNγ** secretion |
|---|---|
| TACI-Mab1 | 3.067 |
| TACI-Mab2 | 9.306 |
| RCT-18 | No inhibitory activity |
| Belimumab | No inhibitory activity |

### Test Example 5: Affinity assay

A certain amount of test sample was subjected to affinity capture by a biosensor chip Protein A (GE, 29127556), then antigens at a series of concentration gradients were allowed to flow on the surface of the chip. The reaction signals were detected in real time by using Biacore (GE, 8K) to obtain association and dissociation curves. After the dissociation was completed for each cycle, the biochip was washed and regenerated with a 10 mM glycine-hydrochloric acid solution at pH 1.5 (GE, BR-1003-54). The experimental data were fitted using BIAevaluation version 4.1, GE software with a 1:1 model to obtain affinity values. The related antigen proteins used in this assay are as follows: human IL-23 (CT048-H08H, Sino biological), human IL-12 (CT050-H08H, Sino biological), human BAFF (10056-HNCH, Sino biological), human APRIL (5860-AP-010/CF, R&D Systems), cynomolgus monkey p40 (10215-CL, R&D Systems), cynomolgus monkey BAFF (BAF-CM412B, Kactus), cynomolgus monkey APRIL (APR-CM410B, Kactus), mouse BAFF (BAF-M521y, Aero Biosystems), and mouse APRIL (7907-AP/CF, R&D Systems).

The experimental results for the TACI antibody fusion proteins constructed by the present disclosure are shown in Tables 28-36 below. The experimental results showed that the antibody fusion proteins constructed by the present disclosure were able to bind with high affinity to human IL-23, human IL-12, human BAFF, human APRIL, cynomolgus monkey p40, cynomolgus monkey BAFF, cynomolgus monkey APRIL, mouse BAFF, and mouse APRIL. The antibody fusion proteins had a stronger affinity for human BAFF and mouse BAFF than the control RCT-18 and belimumab and had cross-binding activity for cynomolgus monkey BAFF and mouse BAFF.

**Table 28. Experimental results for Biacore assay on the TACI antibody fusion proteins and a human BAFF protein**

| Name | Human BAFF | | |
|---|---|---|---|
| | ka (1/Ms) | kd (1/s) | KD (M) |
| TACI-Mab 1 | 1.01E+06 | 9.72E-05 | 9.66E-11 |
| TACI-Mab2 | 2.24E+06 | 5.32E-05 | 2.38E-11 |
| RCT-18 | 9.64E+05 | 1.80E-04 | 1.86E-10 |
| Belimumab | 1.64E+06 | 1.71E-04 | 1.05E-10 |

**Table 29. Experimental results for Biacore assay on the TACI antibody fusion proteins and a mouse BAFF protein**

| Name | Mouse BAFF | | |
|---|---|---|---|
| | ka (1/Ms) | kd (1/s) | KD (M) |
| TACI-Mab 1 | 1.27E+06 | 9.53E-05 | 7.49E-11 |
| TACI-Mab2 | 2.01E+06 | 6.56E-05 | 3.26E-11 |
| RCT-18 | 7.40E+05 | 8.98E-05 | 1.21E-10 |
| Belimumab | 1.38E+06 | 1.44E-04 | 1.04E-10 |

**Table 30. Experimental results for Biacore assay on the TACI antibody fusion proteins and a cynomolgus monkey BAFF protein**

| Name | Cynomolgus monkey BAFF | | |
|---|---|---|---|
| | ka (1/Ms) | kd (1/s) | KD (M) |
| TACI-Mab 1 | 3.03E+05 | 7.81E-05 | 2.58E-10 |
| TACI-Mab2 | 6.33E+05 | 5.91E-05 | 9.33E-11 |
| Belimumab | 4.81E+05 | 2.21E-04 | 4.59E-10 |

**Table 31. Experimental results for Biacore assay on the TACI antibody fusion proteins and a human APRIL protein**

| Name | Human APRIL | | |
|---|---|---|---|
| | ka (1/Ms) | kd (1/s) | KD (M) |
| TACI-Mab 1 | 2.14E+07 | 4.88E-04 | 2.29E-11 |
| TACI-Mab2 | 1.16E+07 | 1.72E-04 | 1.48E-11 |
| RCT-18 | 2.34E+07 | 6.58E-04 | 2.81E-11 |

**Table 32. Experimental results for Biacore assay on the TACI antibody fusion proteins and a cynomolgus monkey APRIL protein**

| Name | Cynomolgus monkey APRIL | | |
|---|---|---|---|
| | ka (1/Ms) | kd (1/s) | KD (M) |
| TACI-Mab 1 | 7.27E+06 | 2.35E-04 | 3.23E-11 |
| TACI-Mab2 | 3.95E+06 | 1.31E-04 | 3.31E-11 |
| RCT-18 | 1.16E+07 | 5.07E-04 | 4.36E-11 |

**Table 33. Experimental results for Biacore assay on the TACI antibody fusion proteins and a mouse APRIL protein**

| Name | Mouse APRIL | | |
|---|---|---|---|
| | ka (1/Ms) | kd (1/s) | KD (M) |
| TACI-Mab 1 | 3.75E+06 | 2.89E-05 | 7.70E-12 |
| TACI-Mab2 | 3.19E+06 | 2.62E-05 | 8.21E-12 |
| RCT-18 | 2.52E+06 | 1.69E-05 | 6.71E-12 |

**Table 34. Experimental results for Biacore assay on the TACI antibody fusion proteins and a human IL-23 protein**

| Name | Human IL-23 | | |
|---|---|---|---|
| | ka (1/Ms) | kd (1/s) | KD (M) |
| TACI-Mab 1 | 1.24E+06 | 8.83E-05 | 7.10E-11 |
| TACI-Mab2 | 1.14E+06 | 1.03E-04 | 9.00E-11 |
| Ustekinumab | 1.26E+06 | 1.07E-04 | 8.44E-11 |

**Table 35. Experimental results for Biacore assay on the TACI antibody fusion proteins and a cynomolgus monkey p40 protein**

| Name | Cynomolgus monkey p40 | | |
|---|---|---|---|
| | ka (1/Ms) | kd (1/s) | KD (M) |
| TACI-Mab1 | 8.91E+05 | 2.38E-04 | 2.67E-10 |
| Ustekinumab | 9.31E+05 | 2.36E-04 | 2.54E-10 |

**Table 36. Experimental results for Biacore assay on the TACI antibody fusion proteins and a human IL-12 protein**

| Abs | Human IL-12 | | |
|---|---|---|---|
| | ka (1/Ms) | kd (1/s) | KD (M) |
| TACI-Mab1 | 4.97E+06 | 2.11E-04 | 4.24E-11 |
| Ustekinumab | 4.66E+06 | 1.80E-04 | 3.87E-11 |

The experimental results for the affinity of representative TACI-Fc fusion proteins of the present disclosure for human BAFF and human APRIL are shown in Tables 37 and 38. The experimental results showed that TACI-9Fc, TACI-9-10Fc, and TACI-9-15Fc constructed by the present disclosure were all able to bind with high affinity to human BAFF and human APRIL.

**Table 37. Experimental results for Biacore assay on the TACI-Fc fusion proteins and a human BAFF protein**

| Name | Human BAFF | | |
|---|---|---|---|
| | ka (1/Ms) | kd (1/s) | KD (M) |
| TACI-9Fc | 2.17E+05 | 1.62E-05 | 7.48E-11 |
| TACI-9-10Fc | 2.34E+05 | 1.89E-05 | 8.10E-11 |
| TACI-9-15Fc | 2.74E+05 | 2.12E-05 | 7.73E-11 |

**Table 38. Experimental results for Biacore assay on the TACI-Fc fusion proteins and a human APRIL protein**

| Name | Human BAFF | | |
|---|---|---|---|
| | ka (1/Ms) | kd (1/s) | KD (M) |
| TACI-9Fc | 1.15E+07 | 2.07E-04 | 1.80E-11 |
| TACI-9-10Fc | 8.75E+06 | 1.12E-04 | 1.28E-11 |
| TACI-9-15Fc | 7.67E+06 | 9.60E-05 | 1.25E-11 |

### Test Example 6: Evaluation of in vivo activity

Three proteins, i.e., chimeric IL-12 (human p40-mouse p35), human IL-23, and human BAFF, were used to simultaneously stimulate the mice to induce the production of cytokines such as IFNγ, TNFα, IL-22, and IgA in mice, and levels of these cytokines were determined to evaluate the *in vivo* activity of the antibodies or fusion proteins.

Female SPF C57BL/6 mice (Zhejiang Vital River Laboratory Animal Technology Co., Ltd., 8 weeks old) were randomly grouped with 5 mice in each group and intraperitoneally injected with three proteins of chimeric IL-12 (2 µg/mouse), human IL-23 (2 µg/mouse), and human BAFF (1 mg/kg) once a day for four days. Test samples (ustekinumab 8 mpk, RCT-18 4 mpk, RCT-18 8 mpk, belimumab 8 mpk, belimumab 16 mpk, TACI-Mab1 4.15 mpk, TACI-Mab1 8.3 mpk, TACI-Mab2 4.45 mpk, and TACI-Mab2 8.9 mpk) were each intraperitoneally injected one hour before the protein injection on the first day and the third day. Plasma samples were collected on the fifth day from each group of mice and tested for levels of IFNy, TNFα, IL-22, and IgA. The source information of assay kits used in this test example is as follows: Mouse IFN-gamma Quantikine ELISA Kit (R&D Systems, MIF00), Mouse TNF-alpha Quantikine ELISA Kit (R&D Systems, MTA00B), Mouse/Rat IL-22 Quantikine ELISA Kit (R&D Systems, M2200), and Mouse IgA ELISA Kit (Abcam, ab157717). RCT-18, belimumab, and ustekinumab were used as positive controls, and PBS was used as a negative control. The drug molar concentrations of TACI-Mab1 8.3 mpk, TACI-Mab2 8.9 mpk, and belimumab 8 mpk were identical to that of RCT-18 4 mpk; the drug molar concentrations of TACI-Mab1 8.3 mpk and TACI-Mab2 8.9 mpk were identical to that of ustekinumab 8 mpk.

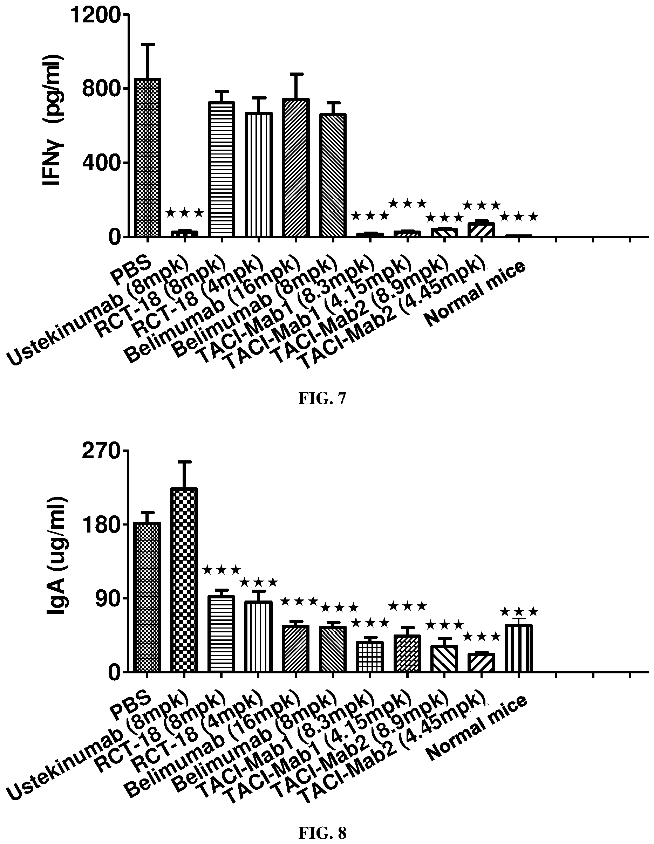

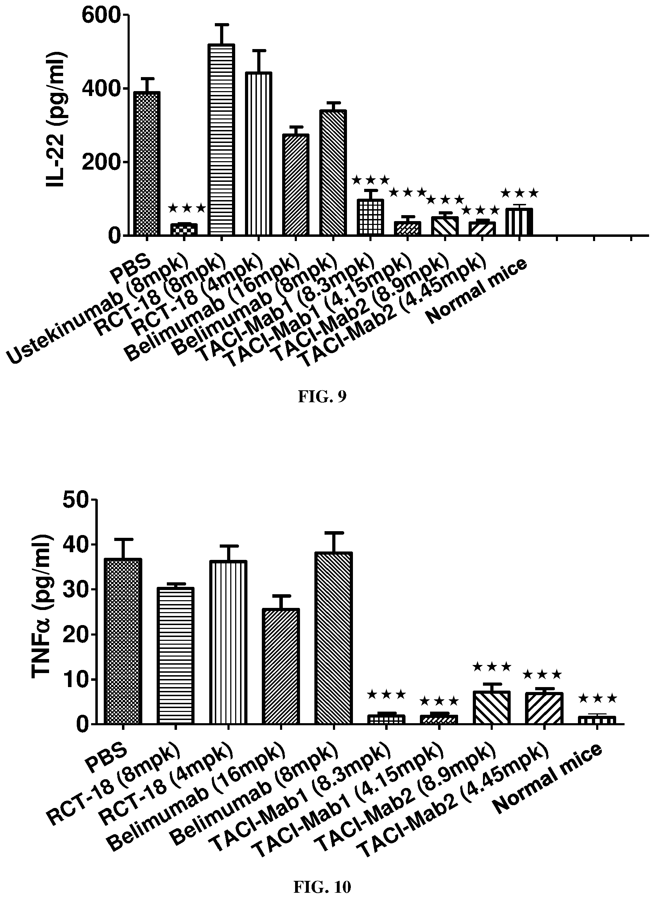

The experimental results are shown in FIGs. 7-10. The experimental results showed that the TACI-Mab1 and TACI-Mab2 antibody fusion proteins of the present disclosure were able to significantly inhibit the secretion of TNFα, IFNγ, and IL-22, while RCT-18 and belimumab were unable to inhibit the secretion of TNFα, IFNγ, and IL-22. In addition, the TACI-Mab1 and TACI-Mab2 antibody fusion proteins were able to significantly inhibit the IgA secretion at both doses and had a stronger inhibitory activity than RCT-18 and belimumab, while ustekinumab was unable to inhibit the IgA secretion.

### Test Example 7: Pharmacokinetic experiment in rats

An *in vivo* pharmacokinetic study was performed in SD rats. Male SD rats (Zhejiang Vital River Laboratory Animal Technology Co., Ltd.) were randomly grouped with 4 rats in each group, and the drugs were administered by intravenous injection. 0.2 mL of whole blood was collected before the administration and 5 min, 8 h, 24 h, 48 h, 84 h, 9 days, 10 days, 14 days, 21 days, and 28 days after the administration. Without anticoagulation, the collected blood was left to stand at 4 °C for 30 min, and centrifuged at 1000 g for 15 min. The upper-layer supernatant was added to an EP tube and stored at -80 °C. The plasma concentration in serum was determined by the ELISA method, and pharmacokinetic parameters and *in vivo* half-life of test drugs were calculated by Winnolin software.

The experimental results for pharmacokinetics of the TACI fusion proteins of the present disclosure in rats are shown in Table 39. The experimental results showed that the TACI fusion proteins of the present disclosure had better stability with an *in vivo* half-life of more than 4 days.

**Table 39. Experimental results for pharmacokinetics of the TACI fusion proteins in rats**

| Sample name | TACI-9-15Fcl | TACI-9-15Fc2 | TACI-9-15Fc3 |
|---|---|---|---|
| Dose iv. | 5 mg/kg | 5 mg/kg | 5 mg/kg |
| Half-life | 4.2 ± 0.3 days | 4.4 ± 0.8 days | 4.2 ± 0.3 days |

The experimental results for pharmacokinetics of the TACI fusion proteins of the present disclosure in rats are shown in Table 40. The experimental results showed that the TACI-Mab 1 and TACI-Mab2 antibody fusion proteins of the present disclosure had a good half-life in rats, with the half-lives of the three parts, the ustekinumab end, the TACI end, and the intact molecule, being all about 9 days; while RCT-18 has a shorter half-life of only about 0.7 days.

**Table 40. Experimental results for pharmacokinetics of the TACI antibody fusion proteins in rats**

| Sample name | TACI-Mab 1 | TACI-Mab2 | RCT-18 |
|---|---|---|---|
| Dose iv. | 12.5 mg/kg | 13.5 mg/kg | 6 mg/kg |
| Ustekinumab end | 9.8 ± 0.6 days | 9.2 ± 2.6 days | / |
| TACI end | 9.0 ± 1.2 days | 9.4 ± 1.4 days | 0.7 ± 0.06 days |
| Intact molecule | 9.3 ± 1.2 days | 9.1 ± 1.8 days | / |

### Test Example 8: Plasma stability test

Test samples subjected to sterile filtration were added to human plasma to make a final concentration of 0.1 mg/mL, and the mixture was incubated at 37 °C for 14 days. The test samples were taken on day 0, day 7, and day 14 and stored at -80 °C for later use. 100 µL of Dynabeads M-280 Streptavidin (Invitrogen, 11206D) was washed three times with 1 mL of PBST (PBS + 0.05% Tween-20), and 1 mL of 0.1% Blocker^{™} Casein (Thermo, 37528) (containing 8 µg of biotin-labeled BAFF) was added. The mixture was mixed homogeneously by turning the tube upside down and incubated at 37 °C for 60 min. The coated Dynabeads M-280 magnetic beads were washed 3 times with 1 mL of PBST, and 50 µL of the test sample (the sample was diluted with 1 mL of 0.1% Blocker^{™} Casein). The mixture was mixed homogeneously by turning the tube upside down at 37 °C for 120 min to capture the sample in plasma. The beads were washed three times with PBST, twice with PBS, and twice with ultrapure water, and 30 µL of Pierce^{™} IgG eluent (Thermo Scientific, 21004) was added. The mixture was mixed homogeneously by turning the tube upside down at room temperature for 6 min. The eluted solution was neutralized by adding 1 M tris-HCl at pH 8.0 and concentrated by rotary evaporation to dryness to remove water. 8 M guanidine hydrochloride was added, and the mixture was incubated at 70 °C for 10 min, then diluted with ultrapure water. The deglycase PNGase F (Biolabs, P0704L) was added, and the mixture was incubated at 37 °C for 2 h. DTT was added, and the mixture was incubated at 70 °C for 10 min. The sample was subjected to cleavage analysis by mass spectrometry.

The results of the plasma stability test are shown in Table 41 below. The test results showed that no cleavage of the TACI-Mab1 and TACI-Mab2 antibody fusion proteins of the present disclosure was detected in human plasma, while RCT-18 showed a gradual increase in the number of cleavage fragments with the prolongation of the incubation time, and the percentage of cleavage molecules was more than 60% by day 14.

**Table 41. Plasma stability test for TACI fusion proteins**

| Days | TACI-Mab1 | TACI-Mab2 | RCT-18 |
|---|---|---|---|
| Day 0 | No cleavage was detected | No cleavage was detected | Percentage of TACI cleavage of 17.51% |
| Day 7 | No cleavage was detected | No cleavage was detected | Percentage of TACI cleavage of 27.10% |
| Day 14 | No cleavage was detected | No cleavage was detected | Percentage of TACI cleavage of 62.59% |

### Test Example 9: Pharmacodynamics in systemic lupus erythematosus animal models

MRL/lpr mice will spontaneously develop symptoms of an autoimmune disease similar to human systemic lupus erythematosus as the weeks of age increase.

Female MRL/lpr mice were purchased from Cavens Laboratory Animal Co., Ltd., and housed at 5 mice/cage in an SPF-grade environment with a temperature of 20-25 °C and a humidity of 40-60 %. Mice were acclimatized in the laboratory environment for at least one week prior to the experiment. When mice reached 10 to 12 weeks of age and had significantly higher urine protein and dsDNA IgG values than the normal control mice, the drugs (RCT-18 8 mpk (i.e., 8 mg/kg for each dose, the same applies hereinafter), TACI-Mab1 8.3 mpk, TACI-Mab 1 16.6 mpk, TACI-Mab2 8.9 mpk, TACI-Mab2 17.8 mpk, and PBS as a negative control) were administered by subcutaneous injection twice a week (once on Monday and once on Thursday) for 8 consecutive weeks. Mice were observed regularly for hair and skin status, urine and plasma were collected, and urine protein content and plasma dsDNA IgG levels were determined. After the experiment was completed, the kidneys of the mice were taken, fixed and stained with H&E to observe the glomerular and tubular lesions and inflammatory cell infiltration of the kidney tissue of mice in each group, and to score the degree of renal lesions. In this test example, the concentration of the urine protein was determined by using the Bradford Protein Assay Kit (purchased from Shanghai Beyotime Biological Tech. Co., Ltd.), as well as Mouse anti-dsDNA IgG-specific ELISA Kit (purchased from Alpha Diagnostic International).

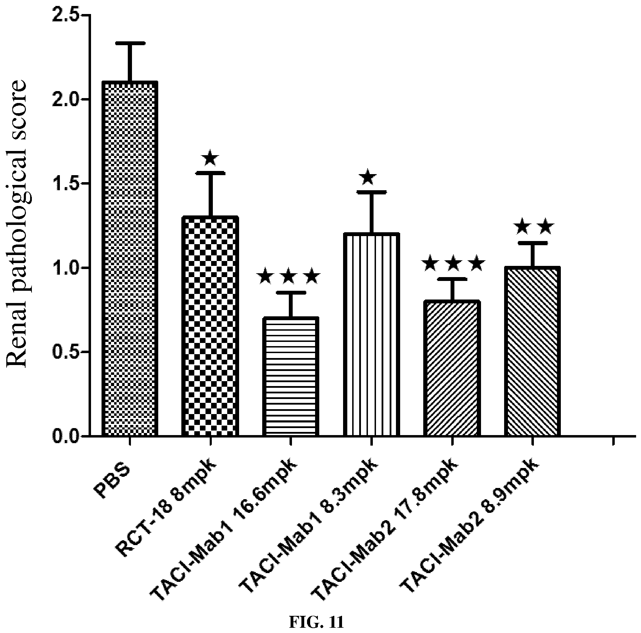

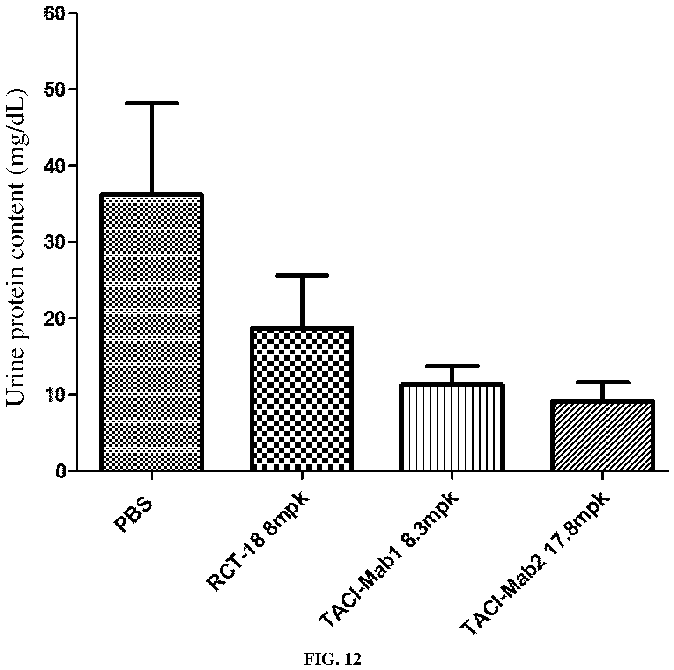

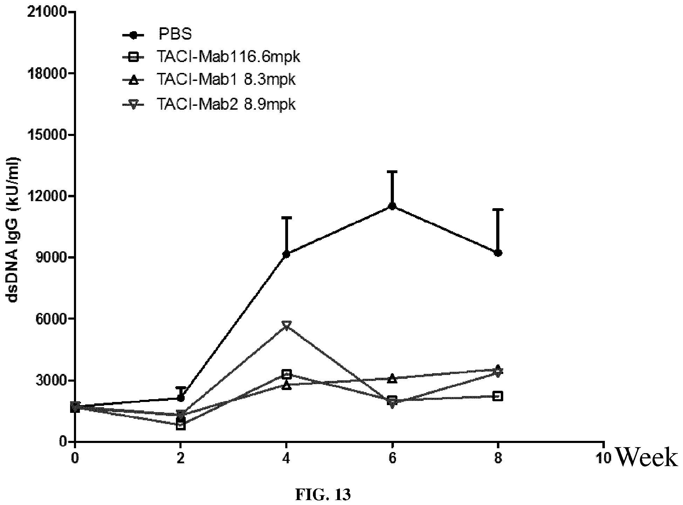

The experimental results are shown in FIGs. 11-13. The experimental results showed that the kidney tissue of the mice in the negative control group had relatively significant inflammatory cell infiltration, and the degree of renal lesions in the TACI-Mab1 and TACI-Mab2 groups of the present disclosure was reduced along with the increase of the dose, showing a certain dose effect. In addition, the TACI-Mab1 8.3 mpk and TACI-Mab2 17.8 mpk of the present disclosure were able to significantly reduce the concentration of the urine in mice; the dsDNA IgG concentration in the TACI-Mab 1 8.3 mpk, TACI-Mab1 16.6 mpk, and TACI-Mab2 8.9 mpk groups were less than that of the negative control group.

## Claims

1. A TACI polypeptide, having a sequence set forth in SEQ ID NO: 8, or being a truncated fragment of SEQ ID NO: 8 or a variant thereof, wherein,
the truncated fragment comprises amino acid residues at positions 48-85 of SEQ ID NO: 8;
the variant is a variant having one or more amino acid replacements at positions selected from the group consisting of positions 49, 52, 53, 57, 65, 82, and 83 in SEQ ID NO: 8 or the truncated fragment thereof, wherein the positions for the amino acid replacements are amino acid residue positions numbered in natural order relative to the sequence SEQ ID NO: 8.

2. A TACI polypeptide, having a sequence set forth in SEQ ID NO: 8, or being a truncated fragment of SEQ ID NO: 8 or a variant thereof, wherein the truncated fragment comprises amino acid residues at positions 51-85 of SEQ ID NO: 8, and the variant is a variant having one or more amino acid replacements at positions selected from the group consisting of positions 52, 53, 57, 65, 82, and 83 in SEQ ID NO: 8 or the truncated fragment thereof, wherein the positions for the amino acid replacements are amino acid residue positions numbered in natural order relative to the sequence SEQ ID NO: 8.

3. The TACI polypeptide according to claim 1 or 2, wherein the truncated fragment of the TACI polypeptide comprises:
amino acid residues at positions 48-86 of SEQ ID NO: 8;
amino acid residues at positions 48-87 of SEQ ID NO: 8;
amino acid residues at positions 48-88 of SEQ ID NO: 8;
amino acid residues at positions 50-85 of SEQ ID NO: 8; or
amino acid residues at positions 49-85 of SEQ ID NO: 8.

4. The TACI polypeptide according to any one of claims 1-3, wherein:
the variant is a variant having one or more amino acid replacements selected from the group consisting of 49T or 49R, 52S, 53E or 53Q, 57E, 65T or 65A, 82A or 82R, and 83Y in a sequence of SEQ ID NO: 8, SEQ ID NO: 10, SEQ ID NO: 13, SEQ ID NO: 14, SEQ ID NO: 15, or SEQ ID NO: 68, or
the variant is a variant having one or more amino acid replacements selected from the group consisting of 52S, 53E or 53Q, 57E, 65T or 65A, 82A or 82R, and 83Y in a sequence of SEQ ID NO: 69 or SEQ ID NO: 70;
wherein the positions for the amino acid replacements are amino acid residue positions numbered in natural order relative to the sequence SEQ ID NO: 8;
preferably, the variant is:
a variant having any one amino acid replacement selected from the group consisting of 49T, 52S, 53E, 53Q, 57E, and 82A in a sequence of SEQ ID NO: 8, SEQ ID NO: 10, SEQ ID NO: 13, SEQ ID NO: 14, SEQ ID NO: 15, or SEQ ID NO: 68;
a variant having any one amino acid replacement selected from the group consisting of 52S, 53E, 53Q, 57E, and 82A in a sequence of SEQ ID NO: 69 or SEQ ID NO: 70;
a variant having amino acid replacements of 49R and 65T in a sequence of SEQ ID NO: 8, SEQ ID NO: 10, SEQ ID NO: 13, SEQ ID NO: 14, SEQ ID NO: 15, or SEQ ID NO: 68;
a variant having amino acid replacements of 49R and 65A in a sequence of SEQ ID NO: 8, SEQ ID NO: 10, SEQ ID NO: 13, SEQ ID NO: 14, SEQ ID NO: 15, or SEQ ID NO: 68;
a variant having amino acid replacements of 49R, 65T, and 82R in a sequence of SEQ ID NO: 8, SEQ ID NO: 10, SEQ ID NO: 13, SEQ ID NO: 14, SEQ ID NO: 15, or SEQ ID NO: 68;
a variant having amino acid replacements of 53E and 57E in a sequence of SEQ ID NO: 8, SEQ ID NO: 10, SEQ ID NO: 13, SEQ ID NO: 14, SEQ ID NO: 15, SEQ ID NO: 68, SEQ ID NO: 69, or SEQ ID NO: 70;
a variant having amino acid replacements of 52S, 53E, and 57E in a sequence of SEQ ID NO: 8, SEQ ID NO: 10, SEQ ID NO: 13, SEQ ID NO: 14, SEQ ID NO: 15, SEQ ID NO: 68, SEQ ID NO: 69, or SEQ ID NO: 70;
a variant having amino acid replacements of 49T and 82A in a sequence of SEQ ID NO: 8, SEQ ID NO: 10, SEQ ID NO: 13, SEQ ID NO: 14, SEQ ID NO: 15, or SEQ ID NO: 68;
a variant having amino acid replacements of 49T and 83Y in a sequence of SEQ ID NO: 8, SEQ ID NO: 10, SEQ ID NO: 13, SEQ ID NO: 14, SEQ ID NO: 15, or SEQ ID NO: 68;
a variant having amino acid replacements of 49T, 82A, and 83Y in a sequence of SEQ ID NO: 8, SEQ ID NO: 10, SEQ ID NO: 13, SEQ ID NO: 14, SEQ ID NO: 15, or SEQ ID NO: 68; or
a variant having amino acid replacements of 49T, 53E, 57E, and 82A in a sequence of SEQ ID NO: 8, SEQ ID NO: 10, SEQ ID NO: 13, SEQ ID NO: 14, SEQ ID NO: 15, or SEQ ID NO: 68;
wherein the positions for the amino acid replacements are amino acid residue positions numbered in natural order relative to the sequence SEQ ID NO: 8.

5. The TACI polypeptide according to any one of claims 1-4, wherein the TACI polypeptide has a sequence set forth in any one of SEQ ID NO: 10, SEQ ID NOs: 13-15, SEQ ID NOs: 18-35, and SEQ ID NOs: 68-70; preferably, the TACI polypeptide has a sequence set forth in SEQ ID NO: 32, SEQ ID NO: 33, SEQ ID NO: 34, or SEQ ID NO: 35.

6. A TACI fusion protein comprising the TACI polypeptide according to any one of claims 1-5.

7. The TACI fusion protein according to claim 6, comprising:
A) the TACI polypeptide according to any one of claims 1-5; and
B) an Fc region.

8. The TACI fusion protein according to claim 7, wherein the Fc region is an Fc region of human IgG1;
preferably, the Fc region comprises a first subunit and a second subunit capable of associating with each other, wherein the first subunit and the second subunit have one or more amino acid substitutions for reducing homodimerization; or
the first subunit and/or the second subunit of the Fc region comprises one or more amino acid substitutions, wherein the amino acid substitutions are capable of reducing the binding of the Fc region to an Fc receptor, and preferably, the amino acid substitutions are capable of reducing binding of the Fc region to an Fcy receptor;
more preferably, the Fc region has a sequence set forth in SEQ ID NO: 11, SEQ ID NO: 61, SEQ ID NO: 62, or SEQ ID NO: 63, or a sequence having at least 90% sequence identity to SEQ ID NO: 11, SEQ ID NO: 61, SEQ ID NO: 62, or SEQ ID NO: 63.

9. The TACI fusion protein according to any one of claims 6-8, being a dimeric protein and comprising a polypeptide chain selected from the group consisting of polypeptide chains shown in a, b, and c:
a. from the N-terminus to the C-terminus: [TACI polypeptide]-[linker]-[subunit of Fc region];
b. from the N-terminus to the C-terminus: [subunit of Fc region]-[linker]-[TACI polypeptide]; and
c. from the N-terminus to the C-terminus: [TACI polypeptide 1]-[linker 1]-[subunit of Fc region]-[linker 2]-[TACI polypeptide 2], wherein linker 1 and linker 2 may be identical or different, and TACI polypeptide 1 and TACI polypeptide 2 may be identical or different;
wherein preferably, the linker in the polypeptide chain shown in a, b, or c is selected from the group consisting of a linker set forth in SEQ ID NO: 64 or is selected from the group consisting of a (GₓS)_{y} linker, wherein X is an integer selected from the group consisting of 1 to 5, and Y is an integer selected from the group consisting of 0 to 6; preferably, the linker is a linker set forth in SEQ ID NO: 65 or SEQ ID NO: 66;
more preferably, the TACI fusion protein comprises 2 identical polypeptide chains having sequences set forth in any one of SEQ ID NOs: 36-44.

10. A TACI fusion protein, being a fusion protein of TACI and an antibody and comprising a TACI polypeptide and an antibody, wherein:
A. the TACI polypeptide is the TACI polypeptide according to any one of claims 1-5;
and B. the antibody is an anti-IL23 antibody, an anti-IL12 antibody, or an anti-IL23/IL12 p40 subunit antibody.

11. The TACI fusion protein according to claim 10, wherein the antibody is an anti-IL23/IL12 p40 subunit antibody;
preferably, the antibody comprises a heavy chain variable region and a light chain variable region,
wherein the heavy chain variable region comprises a HCDR1, a HCDR2, and a HCDR3 set forth in SEQ ID NO: 52, SEQ ID NO: 53, and SEQ ID NO: 54, respectively; and
the light chain variable region comprises a LCDR1, a LCDR2, and a LCDR3 set forth in SEQ ID NO: 49, SEQ ID NO: 50, and SEQ ID NO: 51, respectively.

12. The TACI fusion protein according to claim 10 or 11, wherein the antibody comprises a heavy chain variable region set forth in SEQ ID NO: 45 and a light chain variable region set forth in SEQ ID NO: 46.

13. The TACI fusion protein according to any one of claims 10-12, wherein the antibody comprises antibody heavy and light chain constant regions;
preferably, the heavy chain constant region is selected from the group consisting of human IgG1, IgG2, IgG3, and IgG4 constant regions, and the light chain constant region is selected from the group consisting of human antibody κ chain and λ chain constant regions;
more preferably, the antibody comprises a heavy chain having at least 85% sequence identity to SEQ ID NO: 47 and a light chain having at least 85% sequence identity to SEQ ID NO: 48;
most preferably, the antibody comprises a heavy chain set forth in SEQ ID NO: 47 and a light chain set forth in SEQ ID NO: 48.

14. The TACI fusion protein according to any one of claims 10-13, comprising a polypeptide chain as described in d or e:
d. a first polypeptide chain comprising [antibody heavy chain]-[linker]-[TACI polypeptide] from the N-terminus to the C-terminus, and
a second polypeptide chain being an antibody light chain;
e. a first polypeptide chain comprising [antibody heavy chain]-[linker]-[TACI polypeptide] from the N-terminus to the C-terminus, and
a second polypeptide chain comprising [TACI polypeptide]-[linker]-[antibody light chain] from the N-terminus to the C-terminus;
wherein preferably, the linker can be selected from the group consisting of a (GₓS)_{y} linker, wherein X is an integer selected from the group consisting of 1 to 5, and Y is an integer selected from the group consisting of 0 to 6; preferably, the linker is a linker set forth in SEQ ID NO: 65, SEQ ID NO: 66, or SEQ ID NO: 67.

15. The TACI fusion protein according to any one of claims 10-14, wherein
the TACI fusion protein comprises two identical first polypeptide chains set forth in SEQ ID NO: 55 and two identical second polypeptide chains set forth in SEQ ID NO: 56; or
the TACI fusion protein comprises two identical first polypeptide chains set forth in SEQ ID NO: 57 and two identical second polypeptide chains set forth in SEQ ID NO: 58.

16. A pharmaceutical composition, comprising:
the TACI polypeptide according to any one of claims 1-5 or the TACI fusion protein according to any one of claims 6-15, and
one or more pharmaceutically acceptable carriers, diluents, or excipients.

17. A nucleic acid molecule encoding the TACI polypeptide according to any one of claims 1-5 or the TACI fusion protein according to any one of claims 6-15.

18. A host cell, comprising the nucleic acid molecule according to claim 17.

19. A method for treating or ameliorating a B cell disorder or an autoimmune disease, comprising the step of: administering to a subject in need thereof a therapeutically effective amount of the TACI polypeptide according to any one of claims 1-5, a therapeutically effective amount of the TACI fusion protein according to any one of claims 6-15, or a therapeutically effective amount of the pharmaceutical composition according to claim 16;
wherein preferably, the B cell disorder or the autoimmune disease is a disease or condition associated with TACI expression;
more preferably, the autoimmune disease is selected from the group consisting of:
systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis;
the B cell disorder is selected from the group consisting of: tumors, chronic leukocytic leukemia, multiple myeloma, non-Hodgkin's lymphoma, post-transplant lymphoproliferative disorder, and light chain gammopathy;
most preferably, the autoimmune disease is systemic lupus erythematosus.
